(19) **Europäisches Patentamt / European Patent Office / Office européen des brevets**

(11) **EP 3 779 450 A1**

(12) **EUROPEAN PATENT APPLICATION**
published in accordance with Art. 153(4) EPC

(43) Date of publication:
**17.02.2021 Bulletin 2021/07**

(21) Application number: **19774196.0**

(22) Date of filing: **14.03.2019**

(51) Int Cl.:
**G01N 33/68** (2006.01)    **A61K 39/395** (2006.01)
**A61K 45/00** (2006.01)    **A61P 35/00** (2006.01)
**A61P 37/04** (2006.01)    **G01N 33/53** (2006.01)
**G01N 33/574** (2006.01)

(86) International application number:
**PCT/JP2019/010601**

(87) International publication number:
**WO 2019/188354 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.03.2018 JP 2018059834**

(71) Applicants:
• **Kyoto University**
  **Kyoto-shi, Kyoto 606-8501 (JP)**
• **Kinki University**
  **Higashi-Osaka-shi**
  **Osaka 577-8502 (JP)**
• **ONO Pharmaceutical Co., Ltd.**
  **Osaka-shi, Osaka 541-8526 (JP)**

• **SYSMEX CORPORATION**
  **Kobe-shi**
  **Hyogo 651-0073 (JP)**

(72) Inventors:
• **HONJO, Tasuku**
  **Kyoto-shi, Kyoto 606-8501 (JP)**
• **CHAMOTO, Kenji**
  **Kyoto-shi, Kyoto 606-8501 (JP)**
• **HAYASHI, Hidetoshi**
  **Sayama-shi, Osaka 589-8511 (JP)**
• **NAKAGAWA, Kazuhiko**
  **Sayama-shi, Osaka 589-8511 (JP)**
• **GOTO, Megumi**
  **Kobe-shi, Hyogo 651-0073 (JP)**
• **UGA, Hitoshi**
  **Kobe-shi, Hyogo 651-0073 (JP)**

(74) Representative: **De Clercq & Partners**
  **Edgard Gevaertdreef 10a**
  **9830 Sint-Martens-Latem (BE)**

(54) **METHOD FOR ASSISTING DETERMINATION OF EFFICACY OF IMMUNE CHECKPOINT INHIBITOR, REAGENT KIT, DEVICE, AND COMPUTER PROGRAM**

(57)    The present invention relates to a method for assisting a determination of an efficacy of an immune checkpoint inhibitor. The present invention also relates to a reagent kit for use in the method. The present invention also relates to a device and a computer program each for determining the efficacy of an immune checkpoint inhibitor.

EP 3 779 450 A1

**Description**

TECHNICAL FIELD

The present invention relates to a method for assisting a determination of an efficacy of an immune checkpoint inhibitor. The present invention also relates to a reagent kit for use in the method. The present invention also relates to a device and a computer program each for determining the efficacy of an immune checkpoint inhibitor.

BACKGROUND ART

On the surfaces of activated T cells, a receptor molecule called "Programmed cell death-1 (PD-1)" is expressed (see Non-Patent Document 1). PD-1 is known as a molecule which can inhibit the excessive activation of T cells to negatively regulate the immune response in a living body. On the surfaces of activated T cells, a molecule called "Cytotoxic T lymphocyte antigen-4 (CTLA-4)" is also expressed. Similar to PD-1, CTLA-4 also has the function to regulate the activation of T cells. On the other hand, in some of local cancer cells, Programmed cell death-ligand 1 (PD-L1) that is a ligand for PD-1 is expressed on the surfaces of the cells. It is known that these local cancer cells can inhibit the activation of T cells through the binding between PD-L1 expressed on the local cancer cells and PD-1 expressed on the T cells to avoid the attack by the T cells. As a result, the cancer can expand in a living body. Actually, the amount of PD-L1 expressed in a cancer local part and the percentage of poor prognosis of cancer patients correlate with each other. Because PD-L1, PD-1 and CTLA-4 are involved in the regulation of immune systems as mentioned above, these molecules are also called "immune checkpoint molecules".

In recent years, research focus was directed to an antibody against an immune checkpoint molecule as a novel cancer therapy agent. A preparation containing the antibody as an active ingredient is called "an immune checkpoint inhibitor". The immune checkpoint inhibitor can promote the activation of T cells through the inhibition of the above-mentioned molecules capable of inhibiting the activation of T cells and, as a result, can enhance the anti-tumor responsiveness of the T cells. In other words, in therapy using an immune checkpoint inhibitor, cancer can be eliminated through the activation of the immunological state of a living body by the administration of the above-mentioned antibody.

PRIOR ART DOCUMENT

NON-PATENT DOCUMENT

Non-Patent Document 1: Agata at. al., Expression of the PD-1 antigen on the surface of stimulated mouse T and B lymphocytes. Int. Immunol., 1996, vol.8, p.765-772

SUMMARY OF THE INVENTION

PROBLEMS TO BE SOLVED BY THE INVENTION

It is a desideratum to develop a test which can predict and determine the efficacy of an immune checkpoint inhibitor with high accuracy. Hitherto, a method for predicting the effectiveness of an anti-PD-1 antibody drug or an anti-PD-L1 antibody drug by immunostaining a tumor tissue collected from a cancer patient and then confirming the ratio of PD-L1-positive tumor cells is known. The present invention addresses the problem of providing a novel method for predicting the efficacy of an immune checkpoint inhibitor.

SOLUTIONS TO THE PROBLEMS

The present invention provides a method for determining the efficacy of an immune checkpoint inhibitor, the method comprising the steps of: measuring a free protein marker in a liquid sample collected from a subject; and determining the efficacy of the immune checkpoint inhibitor in the subject based on the result of the measurement, wherein the free protein marker is at least one selected from free CTLA-4, free PD-1 and free PD-L1.

The present invention also provides a method for treating cancer, the method comprising the steps of: measuring a free protein marker in a liquid sample collected from a cancer patient; determining the efficacy of an immune checkpoint inhibitor in the patient based on the result of the measurement; and administering the immune checkpoint inhibitor to a patient who is determined that the immune checkpoint inhibitor is effective for the patient, wherein the free protein marker is at least one selected from free CTLA-4, free PD-1 and free PD-L1.

The present invention also provides: a pharmaceutical composition for cancer treatment, which comprises an immune checkpoint inhibitor and is characterized by being administered to a cancer patient, wherein it is determined that the administration of the immune checkpoint inhibitor to the cancer patient is effective by the determination method according to the present embodiment; and a cancer therapeutic agent which comprises an immune checkpoint inhibitor and is characterized by being administered to a cancer patient, wherein it is determined that the administration of the immune checkpoint inhibitor to the cancer patient is effective by the determination method according to the present embodiment.

The present invention also provides a method for acquiring a measurement value for a free protein marker, the method comprising the step of acquiring a measurement value for the free protein marker in a liquid sample collected from a subject, wherein the free protein marker is at least one selected from free CTLA-4, free PD-1 and free PD-L1, and it is suggested that the immune checkpoint inhibitor is effective for the subject when the acquired measurement value for the free protein marker is lower than a predetermined threshold value corresponding to the marker.

The present invention also provides a reagent kit for use in a method for assisting the above-mentioned determination, the reagent kit comprising at least one reagent selected from a reagent comprising a substance capable of binding specifically to free CTLA-4, a reagent comprising a substance capable of binding specifically to free PD-1 and a reagent comprising a substance capable of binding specifically to free PD-L1.

The present invention also provides a method for assisting a determination of an efficacy of an immune checkpoint inhibitor, the method comprising the steps of: carrying out immunostaining of a tumor tissue collected from a subject, and calculating the ratio of PD-L1-positive tumor cells; measuring a free protein marker in a liquid sample collected from the subject; and determining the efficacy of the immune checkpoint inhibitor in the subject based on the result of the calculation and the result of the measurement, wherein the free protein marker is at least one selected from free CTLA-4, free PD-1 and free PD-L1.

The present invention also provides a device for determining the efficacy of an immune checkpoint inhibitor, the device comprising a computer that comprises a processor and a memory placed under the control of the processor, the memory recording a computer program for bringing the computer to execute the steps of: acquiring a measurement value for a free protein marker in a liquid sample collected from a subject; and determining the efficacy of the immune checkpoint inhibitor in the subject based on the measurement value for the free protein marker, wherein the free protein marker is at least one selected from free CTLA-4, free PD-1 and free PD-L1.

The present invention also provides a device for acquiring a measurement value for a free protein marker, the device comprising a computer that comprises a processor and a memory placed under the control of the processor, the memory recording a computer program for bringing the computer to execute the step of acquiring a measurement value for a free protein marker in a liquid sample collected from a subject, the free protein marker is at least one selected from free CTLA-4, free PD-1 and free PD-L1, and it is suggested that the immune checkpoint inhibitor is effective for the subject when the acquired measurement value for the free protein marker is lower than a predetermined threshold value corresponding to the marker.

The present invention also provides a computer program for determining the efficacy of an immune checkpoint inhibitor, wherein the computer program is recorded on a computer-readable medium, and is intended to bring the computer to execute the steps of: acquiring a measurement value for a free protein marker in a liquid sample collected from a subject; and determining the efficacy of the immune checkpoint inhibitor in the subject based on the measurement value for the free protein marker, wherein the free protein marker is at least one selected from free CTLA-4, free PD-1 and free PD-L1.

EFFECTS OF THE INVENTION

According to the present invention, it becomes possible to determine whether or not an immune checkpoint inhibitor is effective for a subject prior to the administration of the immune checkpoint inhibitor to the subject.

BRIEF DESCRIPTION OF THE DRAWINGS

Fig. 1A is a schematic illustration showing one example of the reagent kit of the present embodiment.
Fig. 1B is a schematic illustration showing one example of the reagent kit of the present embodiment.
Fig. 1C is a schematic illustration showing one example of the reagent kit of the present embodiment.
Fig. 2 is a schematic illustration showing one example of the device for determining the efficacy of an immune checkpoint inhibitor.
Fig. 3 is a block diagram showing the configuration of the hardware of the determination device shown above.
Fig. 4A is a flow chart of the determination of the efficacy of an immune checkpoint inhibitor using the determination device shown above.
Fig. 4B is a flow chart of the determination of the efficacy of an immune checkpoint inhibitor using the determination device shown above.
Fig. 4C is a flow chart of the determination of the efficacy of an immune checkpoint inhibitor using the determination device shown above.
Fig. 5 is a flow chart showing the processing procedure performed by a device for acquiring a measurement value for a free protein marker.
Fig. 6A is a graph showing the distribution of measurement values (pg/mL) for free PD-1 in plasma before the administration of an immune checkpoint inhibitor in lung cancer patients who are classified into a PD-L1-negative case group.
Fig. 6B is a graph showing the distribution of measurement values (pg/mL) for free CTLA-4 in plasma before the administration of an immune checkpoint inhibitor in lung cancer patients who are classified into a PD-L1-negative case group.
Fig. 6C is a graph showing the distribution of measurement values (pg/mL) for free PD-L1 in plasma before the administration of an immune checkpoint inhibitor in lung cancer patients who are classified into a PD-L1-negative case group.
Fig. 7 is a flow for determining the efficacy of an immune checkpoint inhibitor employing the combination of the determination by the immunohistochemical staining of PD-L1 and the determination based on a measurement value for a free protein marker.
Fig. 8A illustrates survival rate curves showing the correlation between the measurement values for free PD-1 and the overall survival (OS) period in esophageal cancer patients who receive the administration of an immune checkpoint inhibitor.
Fig. 8B illustrates survival rate curves showing the correlation between the measurement values for free CTLA-4 and the overall survival (OS) period in esophageal cancer patients who receive the administration of an immune checkpoint inhibitor.
Fig. 8C illustrates survival rate curves showing the correlation between the measurement values for free PD-L1 and the overall survival (OS) period in esophageal cancer patients who receive the administration of an immune checkpoint inhibitor.
Fig. 9A illustrates survival rate curves showing the correlation between the measurement values for free PD-1 and the overall survival (OS) period in endometrial cancer patients who receive the administration of an immune checkpoint inhibitor.
Fig. 9B illustrates survival rate curves showing the correlation between the measurement values for free CTLA-4 and the overall survival (OS) period in endometrial cancer patients who receive the administration of an immune checkpoint inhibitor.
Fig. 9C illustrates survival rate curves showing the correlation between the measurement values for free PD-L1 and the overall survival (OS) period in endometrial cancer patients who receive the administration of an immune checkpoint inhibitor.

DESCRIPTION OF EMBODIMENTS

[1. Method for determining the efficacy of immune checkpoint inhibitor]

In the method for determining the efficacy of an immune checkpoint inhibitor (also referred to as the "determination method", hereinafter) according to the present embodiment, firstly at least one selected from free PD-1, free CTLA-4 and free PD-L1 is measured as a free protein marker in a liquid sample collected from a subject. In a preferred embodiment, at least two selected from free PD-1, free CTLA-4 and free PD-L1 are measured. In a more preferred embodiment, free PD-1, free CTLA-4 and free PD-L1 are measured.
The immune checkpoint inhibitor may be any one, as long as the immune checkpoint inhibitor is a preparation containing at least one selected from an anti-PD-1 antibody, an anti-CTLA-4 antibody and an anti-PD-L1 antibody as an active ingredient. Known examples of preparations containing an anti-PD-1 antibody as an active ingredient are nivolumab and pembrolizumab. Known examples of preparations containing an anti-PD-L1 antibody as an active ingredient are atezolizumab, avelumab and durvalumab. A known example of a preparation containing an anti-CTLA-4 antibody as an active ingredient is ipilimumab. The determination method of the present embodiment is particularly suitable for the determination of the efficacy of a preparation containing an anti-PD-1 antibody as an active ingredient.
An example of a suitable subject is a person who is suspected to be affected by cancer or a patient who is affected by cancer. The term "cancer patient" as used herein also includes a subject from whom a tumor tissue is removed. wherein certain embodiments an immune checkpoint inhibitor is administered as an adjuvant chemotherapy after the removal of a tumor tissue. The determination method of the present embodiment can also be employed for the determination of the efficacy of an immune checkpoint inhibitor for an adjuvant chemotherapy. A preferred cancer patient is a cancer patient who does not receive a therapy with an immune checkpoint inhibitor yet. The subject may receive a therapy other than the therapy with an immune checkpoint inhibitor. Examples of the therapy include a surgery (operation), a radiotherapy, a chemotherapy and a combination thereof. The type of the cancer is not particularly limited, and examples of the cancer include various types of cancer such as solid cancer and hematologic cancer. Examples of solid cancers include lung cancer, esophageal cancer, endometrial cancer, kidney cancer, ovarian cancer, melanoma, stomach cancer and colorectal cancer. Examples of hematologic cancers include leukemia, malignant lymphoma and multiple myeloma. Among these types of cancer, lung cancer (particularly non-small cell lung cancer), esophageal cancer and endometrial cancer are particularly suitable for the determination method of the present embodiment.

As mentioned above, a method for predicting the effectiveness of an immune checkpoint inhibitor by immunostaining a tumor tissue collected from a cancer patient and then confirming the ratio of PD-L1-positive tumor cells is known. However, a tumor tissue is used in this method, and therefore this method cannot be applied to a subject from whom a tumor tissue cannot be collected because of physical burdens or the like. In contrast, in the determination method of the present embodiment, a sample that is collected by a less invasive manner, such as a blood sample, can be used. Namely, the determination method of the present embodiment can be applied to a subject from whom it is difficult to collect a tumor tissue. Furthermore, immunostaining is not suitable for automatization, because the testing technique for immunostaining is complicated. In contrast, the measurement of a free protein marker in a liquid sample is suitable for automatization, and therefore the determination method of the present embodiment is preferred from the viewpoint of the promotion of the efficiency of a pathological examination.

It is possible to employ the above-mentioned immunostaining and the determination method of the present embodiment in combination. In this case, the accuracy of the determination can be improved. For example, as mentioned in the section "EXAMPLES" below, the determination method of the present embodiment makes it possible to extract patients for whom an immune checkpoint inhibitor is effective among from patients in each of which the ratio of PD-L1-positive tumor cells is smaller than a predetermined value. In this case, the subject is a cancer patient who is determined that the ratio of PD-L1-positive tumor cells is smaller than a predetermined value by the immunostaining of a tumor tissue.

The immunostaining of a tumor tissue can be carried out by an immunohistochemical staining method that is known in the art. Particularly, it is preferred to carry out immunohistochemical staining using an antibody capable of specifically recognizing an immune checkpoint molecule in accordance with the pathology diagnosis guideline or the like. The immunohistochemical staining may be carried out using a commercially available staining kit. Examples of kits suitable for carrying out the immunostaining of PD-L1 in a tumor tissue include PD-L1 IHC 22C3 pharmDx "Dako" (Agilent Technologies, Inc.) and PD-L1 IHC 28-8 pharmDx "Dako" (Agilent Technologies, Inc.).

For example, the PD-L1 immunostaining of a tumor tissue can be carried out in the following manner. First, a tumor tissue is collected from a subject, and the tumor tissue is fixed with 10% neutral buffered formalin within 1 hour. The fixing time is from 12 to 72 hours inclusive. The fixed tumor tissue is dehydrated with ethanol. The dehydrated tumor tissue is immersed in xylene to perform a clearing treatment. The treated tumor tissue is immersed in molten paraffin (60 □ or lower) and is then cooled to produce a formalin-fixed paraffin-embedded (FFPE) tissue specimen. The FFPE tissue specimen is sliced into a section having a thickness of 4 to 5 $\mu$m, and the section is attached onto a glass slide. Xylene is added to the sliced section to carry out a deparaffinization treatment. The deparaffinized sliced section is immersed in ethanol to carry out a hydrophilization treatment. The glass slide having the hydrophilized sliced section attached thereon is placed in a commercially available antigen activation solution and is then heated to carry out an antigen activation treatment. The treated sliced section is subjected to immunohistochemical staining with an anti-PD-L1 antibody to produce a tissue specimen.

The ratio of PD-L1-positive tumor cells is a ratio of the number of tumor cells of which the cell membranes are partially or wholly stained to the number of all of tumor cells in a tissue specimen obtained by immunostaining of a tumor tissue. As the ratio of PD-L1-positive tumor cells, a Tumor Proportion Score (TPS) defined in the staining result determination manual of PD-L1 IHC 22C3 pharmDx "Dako" may be employed. A TPS can be calculated by formula 1 shown below. In the formula, the term "number of all of tumor cells" refers to the number of all of tumor cells in a tissue specimen obtained by the HE staining of a tumor tissue, and is required to be at least 100 cells. The term "number of PD-L1-positive tumor cells" refers to the number of tumor cells in which the cell membranes are partially or completely PD-L1 immunostained in the tissue specimen. In the PD-L1-positive tumor cells, tumor-related immunocytes such as macrophages and lymphocytes are excluded. The number of each of the above-mentioned cells can be counted by using an optical microscope.

[Mathematical formula 1]

$$\text{TPS (\%)} = \frac{\text{Number of PD-L1-positive tumor cell}}{\text{Number of all of tumor cell}} \times 100$$

The "predetermined value" is a cut-off value for the ratio of the PD-L1-positive tumor cells. The predetermined value is not particularly limited, and is appropriately determined depending on the type of the antibody (or staining kit) to be used in the immunostaining, the type of cancer to be examined, and the like. For example, in the case where a tumor tissue of non-small cell lung cancer is immunostained using PD-L1 IHC 22C3pharmDx "Dako", a candidate for a primary therapy with pembrolizumab is determined by employing the TPS of 50% as the predetermined value. A candidate for a secondary therapy with pembrolizumab is determined by employing the TPS of 1% as the predetermined value. More specifically, it is determined as "PD-L1-positive (high expression)" when the TPS is 50% or more, it is determined as "PD-L1-positive (low expression)" when the TPS is 1% or more and less than 50%, and it is determined as "PD-L1-negative (no expression)" when the TPS is less than 1%. A subject who is determined as "PD-L1-positive (high expression)" can become a candidate for a primary therapy with pembrolizumab. A subject who is determined as "PD-L1-positive (low expression)" is excluded from candidates for the primary therapy, but can become a candidate for a secondary therapy with pembrolizumab. A subject who is determined as "PD-L1-negative)" is excluded from candidates for any therapy with pembrolizumab.

The subject in the present embodiment may be a cancer patient having a ratio of PD-L1-positive tumor cells smaller than a predetermined value. Said cancer patient may be a patient who is excluded from candidates for a primary therapy with an immune checkpoint inhibitor.

The liquid sample is not particularly limited, as long as the liquid sample is one collected from a subject and may contain a free protein. Examples of the liquid sample include a blood sample, cerebrospinal fluid, pleural fluid, ascitic fluid, lymphatic fluid and urine. In the present embodiment, a blood sample is preferred as liquid sample. Examples of blood samples include whole blood, plasma and serum, and plasma and serum are particularly preferred blood samples.

In the case where insoluble contaminants such as cells are contained in the liquid sample, the contaminants may be removed from the liquid sample by known means such as centrifugation and filtration. The liquid sample may be diluted with a proper water-based medium, if necessary. The water-based medium is not particularly limited, as long as it does not interfere with below-mentioned measurements. Examples of the water-based medium include water, physiological saline and a buffer solution. The buffer solution is not particularly limited, as long as the buffer solution can exhibit a buffering activity at an almost neutral pH value (e.g., a pH value of 6 to 8 inclusive). Examples of the buffer solution include: Good's buffer such as HEPES, MES, Tris, and PIPES; and phosphate-buffered saline (PBS).

All of free PD-1, free CTLA-4 and free PD-L1 are free proteins. Hereinbelow, free PD-1, free CTLA-4 and free PD-L1 are also referred to as "sPD-1", "sCTLA-4" and "sPD-L1", respectively. The term "free protein" as used herein refers to a protein which is detached from the surface of a cell and is present outside of the cell (i.e., in a liquid sample). The free protein may be any one of a protein which is solubilized in a liquid component (i.e., a liquid phase) in a liquid sample, a protein which is encapsulated in a vesicle and a protein which is present on the surface of a vesicle. The vesicle is not particularly limited, as long as the vesicle is a tiny sac comprising a membrane. The vesicle may contain a liquid phase therein. A preferred example of the vesicle is an extracellular vesicle, such as an exosome, a microvesicle and an apoptotic body.

The means for measuring the free protein marker is not particularly limited, as long as a value which reflects the quantity or concentration of a free protein marker contained in the liquid sample (wherein the value is also referred to as a "marker measurement value" or a "measurement value for the marker", hereinafter) can be acquired. In the present embodiment, it is preferred to employ a method of capturing the marker using a substance capable of binding specifically to the free protein marker. The free protein marker contained in the liquid sample can be measured by detecting the free protein marker captured by the substance by a method known in the art.

Examples of the substance capable of binding specifically to the free protein marker include an antibody and an aptamer. Among these substances, an antibody is particularly preferred. Antibodies respectively against the proteins PD-1, CTLA-4 and PD-L1 are known in the art, and are generally easily available. The antibodies against the free protein markers are not particularly limited, as long as the antibodies can bind specifically to the free protein markers. Examples of the antibody include a monoclonal antibody, a polyclonal antibody and a fragment (e.g., Fab, F(ab')2) of each of these antibodies. A commercially available antibody may also be used.

The method for measuring the free protein marker using an antibody is not particularly limited, and may be selected appropriately from the known immunoassays. In the present embodiment, enzyme-linked immunosorbent assay (ELISA) is preferred, and sandwich ELISA is particularly preferred. As one example of the measurement process, a case where the free protein marker in the liquid sample is measured by sandwich ELISA will be described hereinbelow.

Firstly, a complex including the free protein marker, an antibody for capturing the free protein marker (also referred to as a "capture antibody", hereinafter) and antibody for detecting the free protein marker (also referred to as a "detection antibody", hereinafter) is formed on a solid phase. The complex can be formed by mixing a liquid sample that may contain the free protein marker with the capture antibody and the detection antibody. The complex can be formed on a solid phase by bringing a solution containing the complex into contact with the solid phase on which the capture antibody can be immobilized. Alternatively, it is also possible to use a solid phase on which the capture antibody is immobilized previously. For example, the complex can be formed on a solid phase by bringing the solid phase having the capture antibody immobilized thereon, the liquid sample and the detection antibody into contact with one another. In the case where each of the capture antibody and the detection antibody is a monoclonal antibody, it is preferred that an epitope for the capture antibody and an epitope for the detection antibody are different from each other.

The mode of the immobilization of the capture antibody on the solid phase is not particularly limited. For example, the capture antibody and the solid phase may be bonded to each other directly, or the capture antibody and the solid phase may be bonded to each other indirectly with another substance intercalated therebetween. An example of the direct bonding is physical adsorption. An example of the indirect bonding is the bonding through the combination of biotin and avidin or streptavidin (also referred to as an "avidin compound", hereinafter). In this case, it is possible to modify the capture antibody with biotin previously and bond an avidin compound to the solid phase previously, whereby the capture antibody and the solid phase can be bound to each other indirectly through binding between biotin and the avidin compound.

The material for the solid phase is not particularly limited, and may be selected from an organic polymeric compound, an inorganic compound, a biopolymer and the like. Examples of the organic polymeric compound include latex, polystyrene and polypropylene. Examples of the inorganic compound include a magnetic material (e.g., iron oxide, chromium oxide, ferrite), silica, alumina and a glass. Examples of the biopolymer include insoluble agarose, insoluble dextran, gelatin and cellulose. It is possible to use two or more of these substances in combination. The type of the solid phase is not particularly limited, and examples of the type include a particle, a film, a micro plate, a micro tube and a test tube. Among these types, a particle is preferred, and a magnetic particle is particularly preferred.

In the present embodiment, it is also possible to carry out a B/F (Bound/Free) separation procedure for removing an unreacted free component that is not involved in the formation of the complex at a timing between the step of forming the complex and the step of detecting the complex. The term "unreacted free component" as used herein refers to a component which does not constitute the complex. Examples of the unreacted free component include a capture antibody and a detection antibody each of which has not bind to the free protein marker. The means for the B/F separation is not particularly limited. In the case where the solid phase comprises particles, the B/F separation can be achieved by collecting only the complex-capturing solid phase by centrifugation. In the case where the solid phase is a container such as a micro plate and a micro tube, the B/F separation can be achieved by removing a solution containing the unreacted free component. In the case where the solid phase comprises magnetic particles, the B/F separation can be achieved by removing a solution containing the unreacted free component by suction using a nozzle while magnetically constraining the magnetic particles with a magnet. This is preferred from the viewpoint of automatization. Subsequent to the removal of the unreacted free component, the solid phase having the complex captured thereon may be washed with a proper water-based medium such as PBS.

The measurement value of the free protein marker contained in the liquid sample can be acquired by detecting the complex formed on the solid phase by a method known in the art. For example, in the case where an antibody which is labeled with a labeling substance is used as the detection antibody, the measurement value of the marker in the liquid sample can be acquired by detecting a signal generated from the labeling substance. Alternatively, in the case where a labeling secondary antibody against the detection antibody is used, the measurement value for the marker in the liquid sample can also be acquired in the same manner.

As one example of the method for measuring the free protein marker using the antibody, an immune complex transfer immunoassay as disclosed in Japanese Patent Publication Laid-open No. HI-254868 can be employed.

The wording "detect a signal" as used herein includes, within the scope thereof, to qualitatively detect the presence or absence of a signal, to quantify the intensity of a signal, and to semi-quantitatively detect the intensity of a signal. The term "semiquantitative detection" as used herein refers to the matter that the level of the intensity of a signal is rated in stages, such as "no signal is observed", "signal is weak", "signal is moderate" and "signal is intense". In the present embodiment, it is preferred to detect the intensity of a signal quantitatively or semi-quantitatively.

The labeling substance is not particularly limited. For example, the labeling substance may be a substance which can generate a signal by itself (wherein the substance is also referred to a "signal generating substance", hereinafter), or may be a substance which can catalyze a reaction of the substance with another substance to generate a signal. Examples of the signal generating substance include a fluorescent substance and a radioactive isotope. An example of the substance capable of catalyzing a reaction of the substance with another substance to generate a detectable signal is an enzyme. Examples of the enzyme include alkaline phosphatase, peroxidase, $\beta$-galactosidase, and luciferase. Examples of the fluorescent substance include: a fluorescent dye such as fluorescein isothiocyanate (FITC), rhodamine and Alexa Fluor (registered tradename); and a fluorescent protein such as GFP Examples of the radioactive isotope include $^{125}$I, $^{14}$C and $^{32}$P. Among these labeling substances, an enzyme is preferred, and alkaline phosphatase or peroxidase is particularly preferred.

The method for detecting the signal is known in the art. In the present embodiment, the measurement method may be selected appropriately depending on the type of the signal coming from the labeling substance. For example, in the case where the labeling substance is an enzyme, it is possible to measure a signal, e.g., light and color, generated upon the reaction of the enzyme with a substrate for the enzyme using a known device such as a spectrophotometer.

The substrate for the enzyme can be selected appropriately from known substrates depending on the type of the enzyme to be used. For example, in the case where alkaline phosphatase is used as the enzyme, examples of the substrate for the enzyme include: a chemiluminescent substrate such as CDP-Star (registered trademark) (disodium 4-chloro-3-(methoxyspiro[1,2-dioxetane-3,2'-(5'-chloro)tricyclo[3.3.1.13,7]decan]-4-yl)phenylphosphate) and CSPD (registered tradename) (disodium 3-(4-methoxyspiro[1,2-dioxetane-3,2-(5'-chloro)tricyclo[3.3.1.13,7]decan]-4-yl)phenylphosphate); and a chromogenic substrate such as 5-bromo-4-chloro-3-indolyl phosphoric acid (BCIP), disodium 5-bromo-6-chloro-indolylphosphate and p-nitrophenylphosphoric acid. In the case where peroxidase is used as the enzyme, examples of the substrate include: a chemiluminescent substrate such as luminol and a derivative thereof; and a chromogenic substrate such as 2,2'-azinobis(3-ethylbenzothiazoline-6-sulfonic acid ammonium salt) (ABTS), 1,2-phenylenediamine (OPD) and 3,3',5,5'-tetramethylbenzidine (TMB).

When the labeling substance is a radioactive isotope, radioactive rays signals can be measured using a known device such as a scintillation counter. In the case where the labeling substance is a fluorescent substance, fluorescent light signals can be measured using a known device such as a fluorescence microplate reader. An excitation wavelength and a fluorescence wavelength can be determined appropriately depending on the type of the fluorescent substance to be used.

The result of the detection of the signal can be used as a measurement value for a marker. For example, in the case where it is intended to detect the intensity of a signal quantitatively, a measurement value for the signal intensity or a value acquired from the measurement value can be employed as a measurement value for a marker. Examples of the value acquired from a measurement value for the signal intensity include: a value determined by subtracting a measurement value for a negative control sample or a background value from the measurement value; and a value acquired by assigning the measurement value to a calibration curve. The negative control sample can be selected appropriately, and an example of the negative control sample is a liquid sample collected from a normal person.

In the present embodiment, it is preferred to measure the free protein marker contained in the liquid sample by sandwich ELISA using the capture antibody immobilized on magnetic particles and the detection antibody labeled with a labeling substance. In this case, the measurement may be carried out using a commercially available fully automated immunoassay system such as HISCL series products (manufactured by Sysmex Corporation).

Subsequently, in the determination method of the present embodiment, the efficacy of an immune checkpoint inhibitor in a subject is determined based on the result of the measurement. In the present embodiment, it is preferred to compare a measurement value for the free protein marker acquired by the measurement and a predetermined threshold value corresponding to the marker and then carry out the determination based on the result of the comparison. More specifically, when the measurement value is lower than the predetermined threshold value, it may be determined that the immune checkpoint inhibitor is effective for the subject. When the measurement value is equal to or higher than the predetermined threshold value, it may be determined that the immune checkpoint inhibitor is not effective for the subject.

In the case where a measurement value for at least one of sPD-1, sCTLA-4 and sPD-L1 is acquired, the determination can be carried out in the following manner.

In one embodiment where a measurement value for sPD-1 is acquired, the measurement value for sPD-1 is compared with a threshold value corresponding to sPD-1. When the measurement value for sPD-1 is lower than the threshold value, it may be determined that the immune checkpoint inhibitor is effective for the subject. When the measurement value for sPD-1 is equal to or higher than the threshold value, it may be determined that the immune checkpoint inhibitor is not effective for the subject.

In another embodiment where a measurement value for sCTLA-4 is acquired, the measurement value for sCTLA-4 is compared with a threshold value corresponding to sCTLA-4. When the measurement value for sCTLA-4 is lower than the threshold value, it may be determined that the immune checkpoint inhibitor is effective for the subject. When the measurement value for sCTLA-4 is equal to or higher than the threshold value, it may be determined that the immune checkpoint inhibitor is not effective for the subject.

In still another embodiment where a measurement value for sPD-L1 is acquired, the measurement value for sPD-L1 is compared with a threshold value corresponding to sPD-L1. When the measurement value for sPD-L1 is lower than the threshold value, it may be determined that the immune checkpoint inhibitor is effective for the subject. When the measurement value for sPD-L1 is equal to or higher than the threshold value, it may be determined that the immune checkpoint inhibitor is not effective for the subject.

In the case where measurement values for two or three of sPD-1, sCTLA-4 and sPD-L1 are acquired, when all of the measurement values are respectively lower than predetermined threshold values corresponding to the markers, it may be determined that the immune checkpoint inhibitor is effective for the subject. When at least one of the measurement values for the markers is equal to or higher than the predetermined threshold value corresponding to the marker, it may be determined that the immune checkpoint inhibitor is not effective for the subject. More specifically, the determination can be made as follows.

In one embodiment where measurement values for sPD-1 and sCTLA-4 are acquired, the measurement value for sPD-1 is compared with a first threshold value and the measurement value for sCTLA-4 is compared with a second threshold value. In this example, the first threshold value is one corresponding to sPD-1 and the second threshold value is one corresponding to sCTLA-4. When the measurement value for sPD-1 is lower than the first threshold value and the measurement value for sCTLA-4 is lower than the second threshold value, it may be determined that the immune checkpoint inhibitor is effective for the subject. When the measurement value for sPD-1 is equal to or higher than the first threshold value or the measurement value for sCTLA-4 is equal to or higher than the second threshold value, it may be determined that the immune checkpoint inhibitor is not effective for the subject.

In another embodiment where measurement values for sPD-1 and sPD-L1 are acquired, the measurement value for sPD-1 is compared with a first threshold value and the measurement value for sPD-L1 is compared with a second threshold value. In this embodiment, the first threshold value is one corresponding to sPD-1 and the second threshold value is one corresponding to sPD-L1. When the measurement value for sPD-1 is lower than the first threshold value and the measurement value for sPD-L1 is lower than the second threshold value, it may be determined that the immune checkpoint inhibitor is effective for the subject. When the measurement value for sPD-1 is equal to or higher than the first threshold value or the measurement value for sPD-L1 is equal to or higher than the second threshold value, it may be determined that the immune checkpoint inhibitor is not effective for the subject.

In another embodiment where measurement values for sCTLA-4 and sPD-L1 are acquired, the measurement value for sCTLA-4 is compared with a first threshold value and the measurement value for sPD-L1 is compared with a second threshold value. In this embodiment, the first threshold value is one corresponding to sCTLA-4 and the second threshold value is one corresponding to sPD-L1. When the measurement value for sCTLA-4 is lower than the first threshold value and the measurement value for sPD-L1 is lower than the second threshold value, it may be determined that the immune checkpoint inhibitor is effective for the subject. When the measurement value for sCTLA-4 is equal to or higher than the first threshold value or the measurement value for sPD-L1 is equal to or higher than the second threshold value, it may be determined that the immune checkpoint inhibitor is not effective for the subject.

In another embodiment where measurement values for sPD-1, sCTLA-4 and sPD-L1 are acquired, the measurement value for sPD-1 is compared with a first threshold value, the measurement value for sCTLA-4 is compared with a second threshold value, and the measurement value for sPD-L1 is compared with a third threshold value. In this embodiment, the first threshold value is one corresponding to sPD-1, the second threshold value is one corresponding to sCTLA-4, and the third threshold value is one corresponding to sPD-L1. When the measurement value for sPD-1 is lower than the first threshold value, the measurement value for sCTLA-4 is lower than the second threshold value, and the measurement value for sPD-L1 is lower than the third threshold value, it may be determined that the immune checkpoint inhibitor is effective for the subject. When the measurement value for sPD-1 is equal to or higher than the first threshold value, or the measurement value for sCTLA-4 is equal to or higher than the second threshold value, or the measurement value for sPD-L1 is equal to or higher than the third threshold value, it may be determined that the immune checkpoint inhibitor is not effective for the subject.

In the present embodiment, the result of the comparison between a measurement value for a free protein marker and a threshold value corresponding to the marker may be rated with a score. For example, when an acquired measurement value for a free protein marker is lower than a threshold value corresponding to the marker, the score is rated as "0". When an acquired measurement value for a free protein marker is equal to or higher than a threshold value corresponding to the marker, the score is rated as "1". In the case where measurement values for two or more free protein markers are acquired, the scores for the markers are summed up. For example, in the case where measurement values for sPD-1 and sCTLA-4 are acquired, when both of the measurement value for sPD-1 and the measurement value for sCTLA-4 are respectively lower than threshold values corresponding to these markers, the score is rated as "0". When either one of the measurement value for sPD-1 or the measurement value for sCTLA-4 is equal to or higher than the threshold value corresponding to the marker, the score is rated as "1". When both of the measurement value for sPD-1 and the measurement value for sCTLA-4 are respectively equal to or higher than the threshold values corresponding to the markers, the score is rated as "2". In the case where a measurement value for sPD-L1 is acquired in place of either one of the measurement value for sPD-1 or the measurement value for sCTLA-4, the score can also be calculated in the same manner.

In the case where measurement values for sPD-1, sCTLA-4 and sPD-L1 are acquired, when all of the measurement value for sPD-1, the measurement value for sCTLA-4 and the measurement value for sPD-L1 are respectively lower than threshold values corresponding to these markers, the score is rated as "0". When any one of the measurement value for sPD-1, the measurement value for sCTLA-4 and the measurement value for sPD-L1 is equal to or higher than a threshold value corresponding to the marker, the score is rated as "1". When either two of the measurement value for sPD-1, the measurement value for sCTLA-4 and the measurement value for sPD-L1 are respectively equal to or higher than the threshold values corresponding to these markers, the score is rated as "2". When all of the measurement value for sPD-1, the measurement value for sCTLA-4 and the measurement value for sPD-L1 are respectively equal to or higher than the threshold values corresponding to these markers, the score is rated as "3".

In the present embodiments, when the score is "0", it may be determined that the immune checkpoint inhibitor is effective for the subject. When the score is 1 or more, it may be determined that the immune checkpoint inhibitor is not effective for the subject.

The predetermined threshold value corresponding to each of the markers may be selected appropriately. For example, the predetermined threshold value may be selected based on data for a free protein marker in a liquid sample collected from a cancer patient. For example, the predetermined threshold value may be selected in the following manner. Firstly, a liquid sample is collected from each of a plurality of cancer patients who do not receive the administration of an immune checkpoint inhibitor yet. After the collection of the liquid sample, an immune checkpoint inhibitor is administered to each of the cancer patients and the clinical course of the cancer patients are followed up. The efficacy of the immune checkpoint inhibitor is confirmed based on a known evaluation measure such as the change in tumor size, a progression free survival (PFS) period and an overall survival (OS) period. The liquid samples are classified into samples from patients for whom the immune checkpoint inhibitor is effective and samples from patients for whom the immune checkpoint inhibitor is not effective. With respect to each of the samples, a free protein marker is measured to acquire a measurement value. Among from the acquired measurement values, a value based on which it is possible to distinguish between patients for whom the immune checkpoint inhibitor is effective and patients for whom the immune checkpoint inhibitor is not effective is determined. The determined value is selected as a predetermined threshold value. For the selection of the predetermined threshold value, it is preferred to take sensitivity, degree of specificity, positive predictive value and negative predictive value of the determination into consideration.

The determination method of the present embodiment makes it possible to determine as to whether or not an immune checkpoint inhibitor is effective for a subject prior to the administration of the immune checkpoint inhibitor to the subject. Thus, information for assisting the determination of the efficacy of an immune checkpoint inhibitor can be provided to a physician or the like. In other words, the determination method of the present embodiment can also be called "a method for predicting the efficacy of an immune checkpoint inhibitor".

[2. Method for acquiring measurement value for free protein marker]

A measurement value for a free protein marker which is acquired by the above-mentioned determination method may also be considered as the information relating to the efficacy of an immune checkpoint inhibitor in a subject. Therefore, the present invention also includes, within the scope thereof, a method for acquiring a measurement value for a free protein marker (also referred to as an "acquisition method", hereinafter).

In the acquisition method of the present embodiment, firstly a free protein marker in a liquid sample collected from a subject is measured. The free protein marker is at least one selected from sPD-1, sCTLA-4 and sPD-L1. Preferably, the free protein markers are at least two selected from sPD-1, sCTLA-4 and sPD-L1. More preferably, the free protein markers are sPD-1, sCTLA-4 and sPD-L1. The details about the subject, the liquid sample and the means for measuring the free protein marker(s) are the same as those mentioned with respect to the determination method above. In a preferred embodiment, the subject is a lung cancer patient (particularly a non-small cell lung cancer patient), an esophageal cancer patient or an endometrial cancer patient. The subject may be a cancer patient who already undergoes the removal of a tumor.

In the acquisition method of the present embodiment, the subject may be a cancer patient who is determined that the ratio of PD-L1-positive tumor cells is smaller than a predetermined value by the immunostaining of a tumor tissue. The details about the immunostaining of a tumor tissue, the ratio of PD-L1-positive tumor cells, the predetermined value and the type of the cancer in the subject are the same as those mentioned with respect to the determination method above. In the present embodiment, the subject may be a patient who is excluded from candidates for a primary therapy with an immune checkpoint inhibitor that contains an anti-PD-1 antibody as an active ingredient based on the result of the immunostaining of a tumor tissue.

The measurement value for the free protein marker, which is acquired by the acquisition method of the present embodiment, can be employed as information suggesting the efficacy of the immune checkpoint inhibitor in a subject based on the comparison of the measurement value with the predetermined threshold value corresponding to the marker. For example, when both of the measurement values for the free protein markers are respectively lower than predetermined threshold values corresponding to the markers, the measurement values for the free protein markers suggest that the immune checkpoint inhibitor is effective for the subject.

In one embodiment, when the measurement value for sPD-1 is lower than a threshold value corresponding to sPD-1, the measurement value suggests that the immune checkpoint inhibitor is effective for the subject. In another embodiment, when the measurement value for sCTLA-4 is lower than a threshold value corresponding to sCTLA-4, the measurement value suggests that the immune checkpoint inhibitor is effective for the subject. In still another embodiment, when the measurement value for sPD-L1 is lower than a threshold value corresponding to sPD-L1, the measurement value suggests that the immune checkpoint inhibitor is effective for the subject.

In one embodiment, when the measurement value for sPD-1 is lower than a first threshold value and the measurement value for sCTLA-4 is lower than a second threshold value, the measurement values suggest that the immune checkpoint inhibitor is effective for the subject. In another embodiment, when the measurement value for sPD-1 is lower than a first threshold value and the measurement value for sPD-L1 is lower than a second threshold value, the measurement values suggest that the immune checkpoint inhibitor is effective for the subject. In another embodiment, when the measurement value for sCTLA-4 is lower than a first threshold value and the measurement value for sPD-L1 is lower than a second threshold value, the measurement values suggest that the immune checkpoint inhibitor is effective for the subject.

In one embodiment, when the measurement value for sPD-1 is lower than a first threshold value, the measurement value for sCTLA-4 is lower than a second threshold value and the measurement value for sPD-L1 is lower than a third threshold value, the measurement values suggest that the immune checkpoint inhibitor is effective for the subject. The details about the predetermined threshold value(s) are the same as those mentioned with respect to the determination method above.

In the acquisition method of the present embodiment, the immune checkpoint inhibitor may be a preparation containing at least one selected from an anti-PD-1 antibody, an anti-CTLA-4 antibody and an anti-PD-L1 antibody as an active ingredient. As the preparation containing an anti-PD-1 antibody as an active ingredient, nivolumab and pembrolizumab are publicly known. As the preparation containing an anti-PD-L1 antibody as an active ingredient, atezolizumab, avelumab and durvalumab are publicly known. As the preparation containing an anti-CTLA-4 antibody as an active ingredient, ipilimumab is publicly known.

[3. Reagent kit]

Within the scope of the present invention, a reagent kit for use in the above-mentioned determination method is also included. The reagent kit of the present embodiment includes at least one reagent selected from a reagent containing a substance capable of binding specifically to sPD-1, a reagent containing a substance capable of binding specifically to sCTLA-4 and a reagent containing a substance capable of binding specifically to free PD-L1. It is preferred that the reagent kit includes at least two reagents selected from a reagent containing a substance capable of binding specifically to sPD-1, a reagent containing a substance capable of binding specifically to sCTLA-4 and a reagent containing a substance capable of binding specifically to sPD-L1. It is more preferred that the reagent kit includes all of a reagent containing a substance capable of binding specifically to sPD-1, a reagent containing a substance capable of binding specifically to sCTLA-4 and a reagent containing a substance capable of binding specifically to sPD-L1. Examples of the substance capable of binding specifically to each of the free protein markers include an antibody and an aptamer. Among these substances, an antibody is particularly preferred.

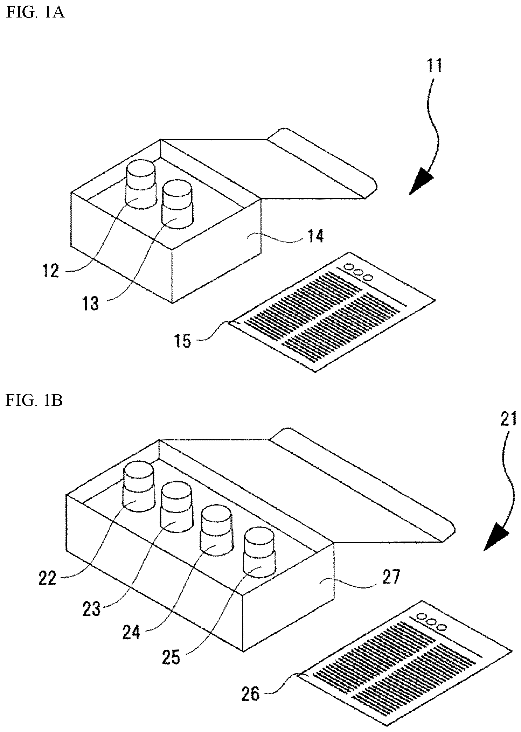

One example of the reagent kit of the present embodiment is shown in Fig. 1A. In Fig. 1A, 11 indicates a reagent kit, 12 indicates a first container which includes a reagent containing a substance capable of binding specifically to free PD-1, 13 indicates a second container which includes a reagent containing a substance capable of binding specifically to free CTLA-4, 14 indicates a packaging box, and 15 indicates a package insert. The reagent kit of this example may also include a third container (not shown) which includes a reagent containing a substance capable of binding specifically to free PD-L1.

In a preferred embodiment, the reagent kit of the present embodiment includes a capture antibody and a detection antibody for each of the free protein markers. The detection antibody may be labeled with a labeling substance. The details about the capture antibody, the detection antibody and the labeling substance are the same as those mentioned with respect to the determination method of the present embodiment above. The reagent kit may also include a solid phase and a substrate. The details about the solid phase and the substrate are the same as those mentioned with respect to the determination method of the present embodiment above.

Another example of the reagent kit of another embodiment is shown in Fig. 1B. In Fig. 1B, 21 indicates a reagent kit, 22 indicates a first container which includes a reagent containing a capture antibody for free PD-1, 23 indicates a second container which includes a reagent containing a labeling antibody for detecting the free PD-1, 24 indicates a third container which includes a reagent containing a capture antibody for free CTLA-4, 25 indicates a fourth container which includes a reagent containing a labeling antibody for detecting free CTLA-4, 26 indicates a package insert, and 27 indicates a packaging box. The reagent kit of this example may also include a fifth container (not shown) which includes a reagent containing a capture antibody for free PD-L1 and a sixth container (not shown) which includes a reagent containing a labeling antibody for detecting free PD-L1 antibody.

In each of the reagent kits, it is preferred to include a calibrator. Examples of the calibrator include: a calibrator for quantifying free PD-1 (a calibrator for PD-1); a calibrator for quantifying free CTLA-4 (a calibrator for CTLA-4); and a calibrator for quantifying free PD-L1 (a calibrator for PD-L1). The calibrator for PD-1 may be provided with, for example, a buffer solution without PD-1 (i.e., a negative control) and a buffer solution containing PD-1 at a known concentration. The calibrator for CTLA-4 may be provided with, for example, a buffer solution without CTLA-4 (i.e., a negative control) and a buffer solution containing CTLA-4 at a known concentration. The calibrator for PD-L1 may be provided with, for example, a buffer solution without PD-L1 (i.e., a negative control) and a buffer solution containing PD-L1 at a known concentration.

Another example of the calibrator is provided with a buffer solution without either one of PD-1, CTLA-4 or PD-L1 (i.e., a negative control), a buffer solution containing PD-1 at a known concentration, a buffer solution containing CTLA-4 at a known concentration and a buffer solution containing PD-L1 at a known concentration. Still another example of the calibrator is provided with a buffer solution without either one of PD-1, CTLA-4 or PD-L1 (i.e., a negative control) and a buffer solution containing two selected from PD-1, CTLA-4 and PD-L1 at known concentrations. Still another example of the calibrator is provided with a buffer solution without either one of PD-1, CTLA-4 or PD-L1 (i.e., a negative control) and a buffer solution containing PD-1, CTLA-4 and PD-L1 at known concentrations.

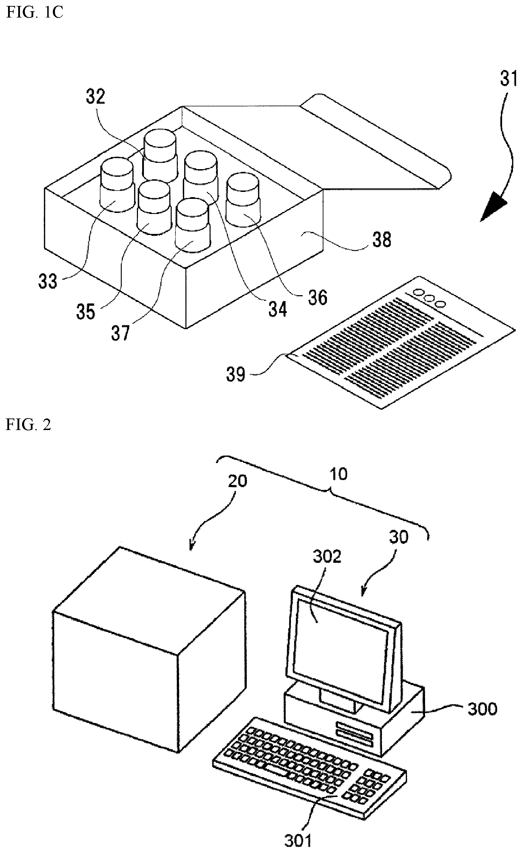

One example of the reagent kit of another embodiment is shown in Fig. 1C. In Fig. 1C, 31 indicates a reagent kit, 32 indicates a first container which includes a reagent containing a capture antibody for free PD-1, 33 indicates a second container which includes a reagent containing a labeling antibody for detecting the free PD-1, 34 indicates a third container which includes a reagent containing a capture antibody for free CTLA-4, 35 indicates a fourth container which includes a reagent containing a labeling antibody for detecting free CTLA-4, 36 indicates a fifth container which includes a buffer solution without either one of PD-1, CTLA-4 or PD-L1, 37 indicates a sixth container which includes a buffer solution containing PD-1 and CTLA-4 at predetermined concentrations, 38 indicates a packaging box, and 39 indicates a package insert. Each of a buffer solution without either one of PD-1, CTLA-4 or PD-L1 and a buffer solution containing PD-1 and CTLA-4 at predetermined concentrations can be used as a calibrator for quantifying PD-1 and CTLA-4. The reagent kit of this embodiment may also include a seventh container (not shown) which includes a reagent containing a capture antibody for free PD-L1, an eighth container (not shown) which includes a reagent containing a labeling antibody for detecting free PD-L1 and a ninth container (not shown) which includes a buffer solution containing PD-L1 at a predetermined concentration (i.e., a calibrator for quantifying PD-L1).

[4. Device and computer program]

Within the scope of the present invention, a device for performing the determination method of the present embodiment is also included. The device is one for determining the efficacy of an immune checkpoint inhibitor (wherein the device is also simply referred to as a "determination device", hereinafter). Within the scope of the present invention, a computer program for allowing a computer to execute the determination method of the present embodiment is also included. The computer program is one for determining the efficacy of an immune checkpoint inhibitor. Within the scope of the present invention, a device for performing the acquisition method of the present embodiment is also included. The device is one for acquiring a measurement value for a free protein marker.

Hereinbelow, one example of the device for performing the method of the present embodiment will be described with reference to drawings. However, the present embodiment is not limited only to the embodiment shown in this example. Fig. 2 is a schematic illustration of the determination device. A determination device 10 shown in Fig. 2 includes an immunoassay device 20 and a computer system 30 connected to the immunoassay device 20. A hardware configuration of the acquisition device for a measurement value for a free protein marker is the same as that of the determination device 10.

In the present embodiment, the immunoassay device is not particularly limited, and may be selected appropriately depending on the mode of the free protein marker measurement method to be employed. In the example shown in Fig. 2, the immunoassay device 20 is a commercially available automatic immunoassay device which can detect a chemiluminescent signal generated by sandwich ELISA using magnetic particles having a capture antibody immobilized thereon and an enzymatically labeled detection antibody. The immunoassay device 20 is not particularly limited as long as the detection of a signal based on the labeling substance used, and may be selected appropriately depending on the type of the labeling substance.

Upon the setting of a reagent containing magnetic particles having a capture antibody immobilized thereon, a reagent containing an enzymatically labeled detection antibody and a liquid sample collected from a subject to the immunoassay device 20, the immunoassay device 20 executes an antigen-antibody reaction using the reagents to acquire a chemiluminescent signal as optical information based on the enzymatically labeled antibody that is bonded specifically to the free protein marker and sends the optical information to a computer system 30.

The computer system 30 includes a computer main body 300, an input section 301 and a display section 302 for displaying sample information, determination results and more. The computer system 30 receives the optical information from the immunoassay device 20. A processor in the computer system 30 executes a computer program for the determination of the efficacy of an immune checkpoint inhibitor, which is installed in a hard disk 313, based on the optical information. The computer system 30 may be a device that is different from the immunoassay device 20 as shown in Fig. 2 or may be a device including the immunoassay device 20 therein. In the latter case, the computer system 30 may work as the determination device 10 by itself. It is also possible to install a computer program for the determination of the efficacy of an immune checkpoint inhibitor on a commercially available automatic immunoassay device.

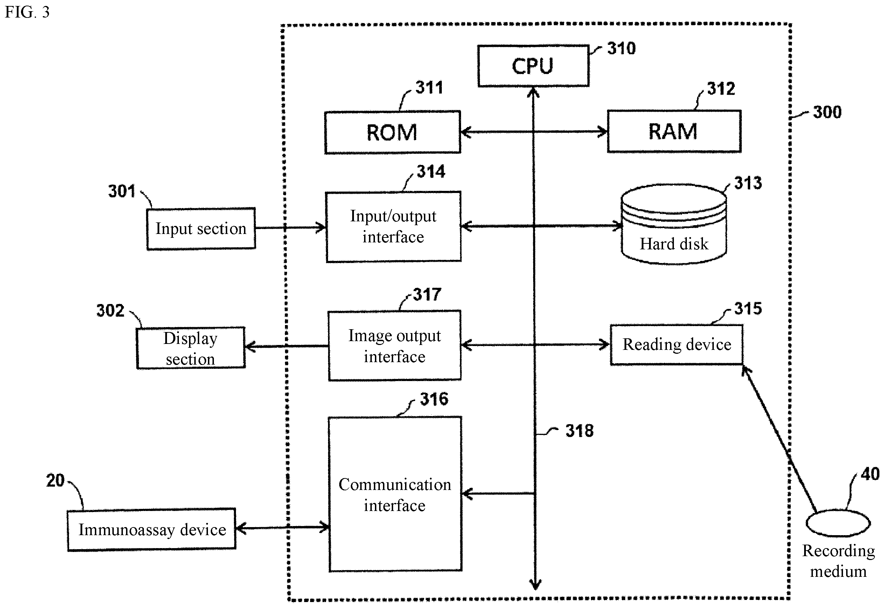

Referring to Fig. 3, the computer main body 300 is provided with a CPU (Central Processing Unit) 310, a ROM (Read Only Memory) 311, a RAM (Random Access Memory) 312, a hard disk 313, an input/output interface 314, a reading device 315, a communication interface 316 and an image output interface 317. The CPU 310, the ROM 311, the RAM 312, the hard disk 313, the input/output interface 314, the reading device 315, the communication interface 316 and the image output interface 317 are connected in a data-communicable manner via a bus 318. The immunoassay device 20 is connected to the computer system 30 in a communicable manner via the communication interface 316.

The CPU 310 can execute a program stored in the ROM 311 or the hard disk 313 and a program loaded in the RAM 312. The CPU 310 calculates measurement values for free protein markers, reads out a predetermined threshold value corresponding to each of the markers which is stored in the ROM 311 or the hard disk 313, and determines as to whether or not an immune checkpoint inhibitor is effective for a subject. The CPU 310 outputs the result of the determination, and allows the display section 302 to display the result.

The ROM 311 is composed of a mask ROM, a PROM, an EPROM, an EEPROM and others. In the ROM 311, a computer program that can be executed by the CPU 310 and data that can be used for the execution of the computer program are recorded. In the ROM 311, data to be used for the below-mentioned determination flow, such as a predetermined threshold value corresponding to each free protein marker, may also be recorded.

The RAM 312 is composed of an SRAM, a DRAM and others. The RAM 312 is used for the read out of the programs recorded on the ROM 311 and the hard disk 313. The RAM 312 can also be used as a workspace for the CPU 310 upon the execution of these programs.

In the hard disk 313, an operating system that is to be executed by the CPU 310, a computer program such as an application program (e.g., a computer program for the above-mentioned cancer determination) and data to be used for the execution of the computer program are installed. In the hard disk 313, data to be used for the below-mentioned determination flow chart, such as a predetermined threshold value corresponding to each free protein marker, may also be recorded.

The reading device 315 is composed of a flexible disk drive, a CD-ROM drive, a DVD-ROM drive and others. The reading device 315 can read out a program or data recorded on a portable recording medium 40.

The input/output interface 314 is composed of a serial interface such as USB, IEEE1394 and RS-232C, a parallel interface such as SCSI, IDE and IEEE1284, and an analogue interface composed of a D/A converter and an A/D converter. To the input/output interface 314, an input section 301 such as a key board and a mouse is connected. An operator can input various commands to the computer main body 300 by means of the input section 301.

One example of the communication interface 316 is Ethernet (registered tradename) interface. The computer main body 300 can also send printing data to a printer or the like by means of the communication interface 316.

The image output interface 317 is connected to the display section 302 that is composed of an LCD, a CRT and the like. According to this configuration, the display section 302 can output a video signal corresponding to the image data received from the CPU 310. The display section 302 can display an image (screen) in response to the input video signal.

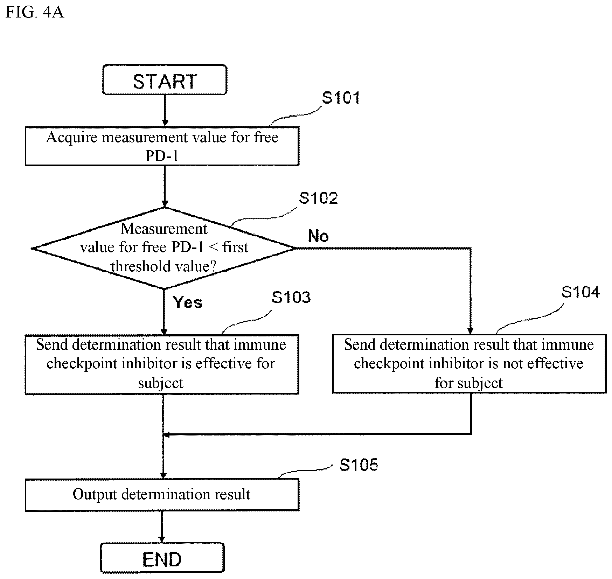

Referring to Fig. 4A, the flow chart for determining the efficacy of an immune checkpoint inhibitor, which is executed by the determination device 10, will be described. In this section, a case where a measurement value for sPD-1 is acquired from a chemoluminescent signal generated by sandwich ELISA using magnetic particles having a capture antibody immobilized thereon and an enzymatically labeled detection antibody, and the determination is carried out using the acquired measurement value will be described as an example. In this example, the first threshold value is a threshold value corresponding to sPD-1. However, the present embodiment is not limited only to this example. It is also possible to acquire a measurement value for sCTLA-4 or a measurement value for sPD-L1 in place of the measurement value for sPD-1.

In step S101, a CPU 310 acquires optical information (a chemoluminescent signal) from an immunoassay device 20, calculates a measurement value for sPD-1 from the acquired optical information, and stores the measurement value in a hard disk 313. In step S102, the CPU 310 compares the calculated measurement value for sPD-1 with the first threshold value stored in the hard disk 313. When the measurement value for sPD-1 is lower than the first threshold value, the processing proceeds to step S103. In step S103, the CPU 310 stores such a determination result that the immune checkpoint inhibitor is effective for a subject in the hard disk 313.

In step S102, on the other hand, when the measurement value for sPD-1 is equal to or higher than the first threshold value, the processing proceeds to step S104. In step S104, the CPU 310 stores such a determination result that the immune checkpoint inhibitor is not effective for the subject in the hard disk 313. In step S105, the CPU 310 outputs the determination result and allows the determination result to be displayed on the display section 302 or allows a printer to print the determination result. In this manner, the information for assisting the determination of the efficacy of an immune checkpoint inhibitor can be provided to a physician or the like.

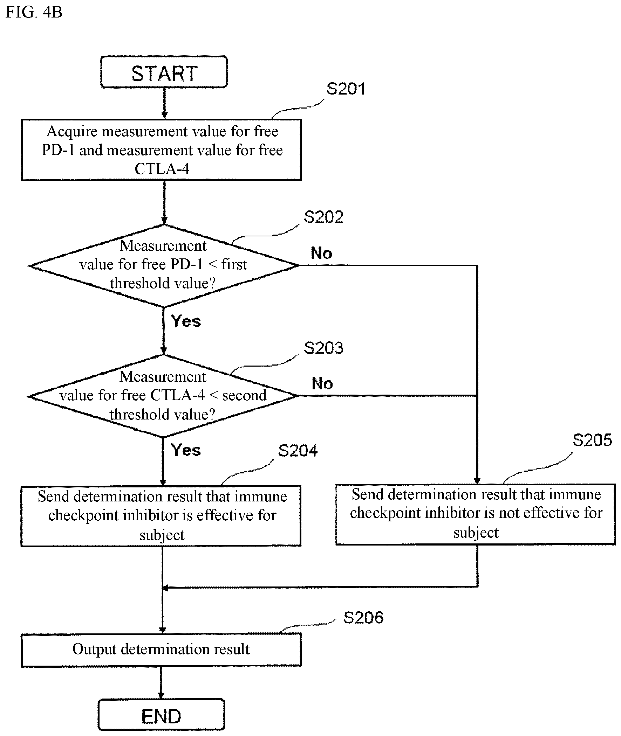

Referring to Fig. 4B, the flow chart for determining the efficacy of an immune checkpoint inhibitor, which is executed by the determination device 10, will be described. In this section, a case where measurement values for sPD-1 and sCTLA-4 are acquired from chemoluminescent signals generated by sandwich ELISA using magnetic particles having a capture antibody immobilized thereon and an enzymatically labeled detection antibody and the determination is carried out using the acquired measurement values will be described as an example. In this example, the first threshold value is one corresponding to sPD-1 and the second threshold value is one corresponding to sCTLA-4. However, the present embodiment is not limited only to this example. It is also possible to acquire a measurement value for sPD-L1 in place of either one of the measurement value for sPD-L1 or the measurement value for and sCTLA-4.

In step S201, a CPU 310 acquires optical information (a chemoluminescent signal) from an immunoassay device 20, calculates measurement values for sPD-1 and sCTLA-4 from the acquired optical information, and stores the measurement values in a hard disk 313. In step S202, the CPU 310 compares the calculated measurement value for sPD-1 with a first threshold value stored in the hard disk 313. When the measurement value for sPD-1 is lower than the first threshold value, the processing proceeds to step S203. In step S203, the CPU 310 compares the calculated measurement value for sCTLA-4 with a second threshold value stored in the hard disk 313. When the measurement value for sCTLA-4 is lower than the second threshold value, the processing proceeds to step S204. In step S204, the CPU 310 stores such a determination result that an immune checkpoint inhibitor is effective for a subject in the hard disk 313.

In step S202, on the other hand, when the measurement value for sPD-1 is equal to or higher than the first threshold value, the processing proceeds to step S205. In step S203, when the measurement value for sCTLA-4 is equal to or higher than the second threshold value, the processing proceeds to step S205. In step S205, the CPU 310 stores such a determination result that the immune checkpoint inhibitor is not effective for the subject in the hard disk 313. In step S206, the CPU 310 outputs the determination result and allows the determination result to be displayed on a display section 302 or allows a printer to print the determination result. In this manner, the information for assisting the determination of the efficacy of an immune checkpoint inhibitor can be provided to a physician or the like. In this example, it is possible to interchange the processing order between step S202 and step S203.

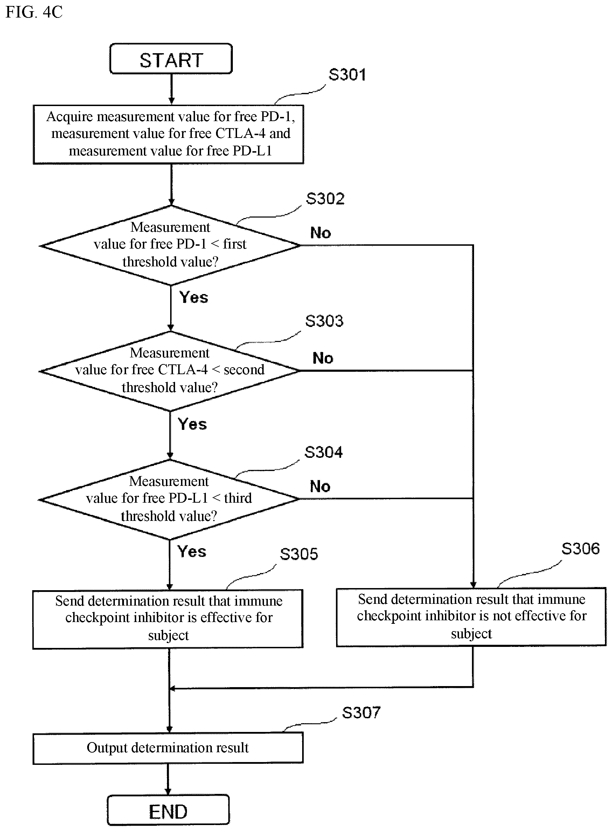

Referring to Fig. 4C, the flow for determining the efficacy of an immune checkpoint inhibitor, which is executed by the determination device 10, will be described. In this section, a case where a measurement value for sPD-L1 is acquired in addition to measurement values for sPD-1 and sCTLA-4 and the determination is carried out using the acquired measurement values will be described as an example. In this embodiment, the first threshold value is one corresponding to sPD-1, the second threshold value is one corresponding to sCTLA-4, and the third threshold value is one corresponding to sPD-L1. However, the present embodiment is not limited only to this example.

In step S301, a CPU 310 acquires optical information (a chemoluminescent signal) from an immunoassay device 20, calculates measurement values for sPD-1, sCTLA-4 and sPD-L1 from the acquired optical information, and stores the measurement values in a hard disk 313. In step S302, the CPU 310 compares the calculated measurement value for sPD-1 with a first threshold value stored in the hard disk 313. When the measurement value for sPD-1 is lower than the first threshold value, the processing proceeds to step S203. In step S303, the CPU 310 compares the calculated measurement value for sCTLA-4 with a second threshold value stored in the hard disk 313. When the measurement value for sCTLA-4 is lower than the second threshold value, the processing proceeds to step S304. In step S304, the CPU310 compares the calculated measurement value for sPD-L1 with a third threshold value stored in the hard disk 313. When the measurement value for sPD-L1 is lower than the third threshold value, the processing proceeds to step S305. In step S305, the CPU 310 stores such a determination result that an immune checkpoint inhibitor is effective for a subject in a hard disk 313.

In step S302, on the other hand, when the measurement value for sPD-1 is equal to or higher than the first threshold value, the processing proceeds to step S306. In step S303, when the measurement value for sCTLA-4 is equal to or higher than the second threshold value, the processing proceeds to step S306. In step S304, when the measurement value for sPD-L1 is equal to or higher than the third threshold value, the processing proceeds to step S306. In step S306, the CPU 310 stores such a determination result that the immune checkpoint inhibitor is not effective for the subject in the hard disk 313. In step S307, the CPU 310 outputs the determination result and allows the determination result to be displayed on a display section 302 or allows a printer to print the determination result. In this example, it is possible to interchange the processing order among step S302, step S303 and step S304.

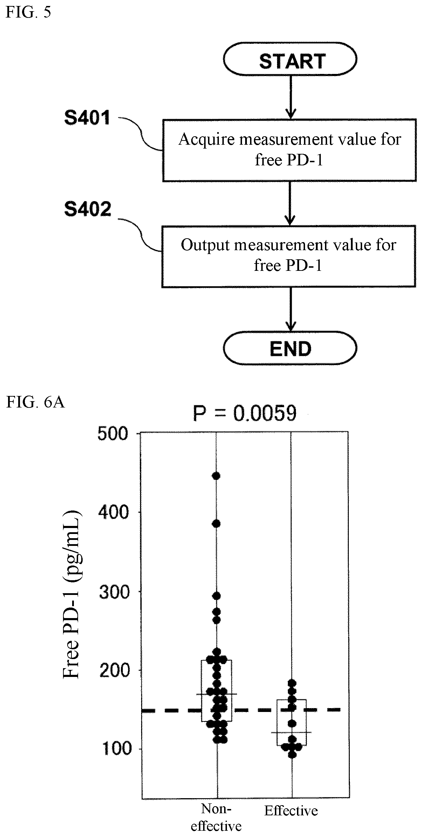

Referring to Fig. 5, a processing procedure which is executed by a device 10 for acquiring a measurement value for a free protein marker will be described. In this section, a case where a measurement value for sPD-1 is acquired from a chemoluminescent signal generated by sandwich ELISA using magnetic particles having a capture antibody immobilized thereon and an enzymatically labeled detection antibody, and the determination is carried out using the acquired measurement value will be described as an example. However, the present embodiment is not limited only to this example. It is also possible to acquire a measurement value for sCTLA-4 or a measurement value for sPD-L1 in place of the measurement value for sPD-1. In another embodiment, it is also possible to acquire measurement values for two free protein markers selected from sPD-1, sCTLA-4 and sPD-L1. It is also possible to acquire measurement values for all of sPD-1, sCTLA-4 and sPD-L1.

In step S401, a CPU 310 acquires optical information (a chemoluminescent signal) from an immunoassay device 20, calculates a measurement value for sPD-1 from the acquired optical information, and stores the measurement value in a hard disk 313. In step S402, the CPU 310 outputs the acquired measurement value for sPD-1 and allows the measurement value to be displayed on a display section 302 or allows a printer to print the measurement value. In this example, it is also possible to output the predetermined threshold values corresponding to the free protein markers together with the measurement values for the markers.

The measurement value for the free protein marker, which is acquired by the acquisition device of the present embodiment, can serve as information suggesting the efficacy of the immune checkpoint inhibitor in a subject based on the comparison of the measurement value with the predetermined threshold value corresponding to the marker. The details are the same as those mentioned with respect to the acquisition method of the present embodiment.

The wording "an immune checkpoint inhibitor is effective for a subject" as used herein refers to the matter that "the immune checkpoint inhibitor is more likely to be effective for the subject", and can also be said that "the subject can be selected as a candidate for the administration of the immune checkpoint inhibitor". The wording "an immune checkpoint inhibitor is not effective for a subject" as used herein refers to the matter that "the immune checkpoint inhibitor is less likely to be effective for the subject", and can also be said that "the subject cannot be selected as a candidate for the administration of the immune checkpoint inhibitor".

[4. Method for treating cancer and pharmaceutical composition]

One embodiment of the present invention relates to a method for treating cancer. The method for treating cancer according to the present embodiment includes the step of administering an immune checkpoint inhibitor to a cancer patient based on the result of the measurement of a free protein marker in a liquid sample collected from the cancer patient. In this treatment method, the free protein marker is at least one selected from free PD-1, free CTLA-4 and free PD-L1. Preferably, the free protein markers are at least two selected from sPD-1, sCTLA-4 and sPD-L1. More preferably, the free protein markers are sPD-1, sCTLA-4 and sPD-L1. The immune checkpoint inhibitor may be a preparation containing at least one selected from an anti-PD-1 antibody, an anti-CTLA-4 antibody and an anti-PD-L1 antibody as an active ingredient. Preferably, the immune checkpoint inhibitor may be a preparation containing an anti-PD-1 antibody as an active ingredient. In one embodiment, the immune checkpoint inhibitor is nivolumab or pembrolizumab.

In the treatment method of the present embodiment, the administration step can be applied to a patient who is determined as a patient for whom the immune checkpoint inhibitor is effective from the result of the measurement of the free protein marker. The details about the determination of the efficacy of the immune checkpoint inhibitor in this method are the same as those mentioned with respect to the determination method above. The details about the type of the cancer, the liquid sample, the means for measuring a free protein marker, the determination and the like are also the same as those mentioned with respect to the determination method above.

The treatment method of the present embodiment is suitable for a lung cancer patient, an esophageal cancer patient and an endometrial cancer patient. In the administration step, it is preferred to administer an immune checkpoint inhibitor in a therapeutically effective amount to a cancer patient. The therapeutically effective amount can be determined appropriately depending on the type of the cancer, the degree of progression of the cancer, the type of the immune checkpoint inhibitor, the therapy guideline or the like.

One embodiment of the present invention relates to a pharmaceutical composition for cancer treatment, which contains an immune checkpoint inhibitor and is administered to a cancer patient who is determined that the immune checkpoint inhibitor is effective by the above-mentioned determination method. In the present embodiment, the pharmaceutical composition for cancer treatment may be composed only of an immune checkpoint inhibitor. The details about the immune checkpoint inhibitor are as mentioned above. The pharmaceutical composition for cancer treatment may contain a pharmaceutically acceptable component. The component can be selected appropriately from pharmaceutical additives that are known in the art. The pharmaceutical composition for cancer treatment may further contain a cancer treatment drug that is different from the immune checkpoint inhibitor. Examples of the cancer treatment drug include a chemotherapy agent and a molecular targeted drug. The form of the pharmaceutical composition for cancer treatment is not particularly limited, and may be a solid form such as crystals and a powder, or may be a liquid such as a solution, an emulsion and a suspension.

Hereinafter, the present invention will be described in more detail with reference to examples. However, the present invention is not limited to these examples.

EXAMPLES

Example 1

With respect to a non-small cell lung cancer patient, the relationship between the concentration of a free protein marker in a blood sample before the administration of an immune checkpoint inhibitor and the therapeutic effect after the administration of the immune checkpoint inhibitor was examined.

1. Subjects

Non-small cell lung cancer patients (50 cases) who did not receive the administration of an immune checkpoint inhibitor were employed as subjects. In Example 1, nivolumab (Ono Pharmaceutical Co., Ltd.) that is an immune checkpoint inhibitor was administered to all of the patients (50 cases), and the patients were followed up by employing a progression free survival (PFS) as an evaluation measure.

2. Liquid samples

Blood was collected from each of the patients (50 cases) before administration of the immune checkpoint inhibitor. The blood thus obtained was separated by the conventional manner to obtain plasma. The plasma samples (50 specimens) were used as liquid samples.

3. PD-L1 immunostaining of tumor tissues

A tumor tissue were collected from each of the patients (50 cases) before the administration of the immune checkpoint inhibitor by surgery or biopsy to prepare a FFPE tissue specimen. The FFPE tissue specimen was sliced, and a sliced section obtained was subjected to a deparaffinization treatment, a hydrophilization treatment and an antigen activation treatment by the conventional manner. The treated sliced section was subjected to immunohistochemical staining with an anti-PD-L1 antibody (28-8 antibody) to produce a tissue specimen. The tissue specimen was observed with an optical microscope, and the number of tumor cells in each of which the cell membrane was partially or wholly stained was counted as the number of "PD-L1-positive tumor cells". A TPS was calculated as the ratio of the PD-L1-positive tumor cells in accordance with the above-shown formula. As a result, it was demonstrated that the TPS was 50% or more in 13 cases among the 50 cases. In the remaining 37 cases, the TPS was less than 50%. Hereinafter, the patients in each of whom the TPS was less than 50% were also referred to as "PD-L1-negative cases".

4 Measurement of free protein marker

(4.1) Preparation of reagents

(4.1.1) First reagent (biotin-labeled antibody solution)

- First reagent for capturing PD-1

As a capture antibody, anti-PD-1 antibody (clone No. MIH4) was used. The antibody was labeled with biotin using Biotin Labeling Kit-SH (Catalog No. LK10, Dojindo Molecular Technologies, Inc.). The concrete operations of the procedure were carried out in accordance with the kit manual. The biotin-labeled anti-PD-1 antibody was added to 100 mM of HEPES (pH 7.5) to prepare a first reagent for capturing PD-1 (the concentration of the antibody: 5 $\mu$g/ml).

- First reagent for capturing CTLA-4

As a capture antibody, anti-CTLA-4 antibody (clone No. BNI3) was used. The antibody was labeled with biotin in the same manner as mentioned above. The biotin-labeled anti-CTLA-4 antibody was added to 100 mM of MES (pH 6.5) to prepare a first reagent for capturing CTLA-4 (the concentration of the antibody: 5 $\mu$g/ml).

- First reagent for capturing PD-L1

As a capture antibody, anti-PD-L1 antibody (clone No. 27A2) was used. The antibody was labeled with biotin in the same manner as mentioned above. The biotin-labeled anti-PD-L1 antibody was added to 100 mM of MES (pH 6.5) to prepare a first reagent for capturing PD-L1 (the concentration of the antibody: 5 $\mu$g/ml).

(4.1.2) Second reagent (solution containing streptavidin-conjugated particles)

Magnetic particles each having streptavidin immobilized on the surface thereof (average particle diameter: 2 $\mu$m; the amount of streptavidin per 1 g of the magnetic particles: 2.9 to 3.5 mg; also referred to as "STA-conjugated magnetic particles", hereinafter) were washed three times with 10 mM of HEPES buffer solution (pH 7.5). The washed STA-conjugated magnetic particles were added to 10 mM of HEPES (pH 7.5) in such a manner that the concentration of streptavidin became 18 to 22 $\mu$g/ml (the concentration of the STA-conjugated magnetic particles became 0.48 to 0.52 mg/ml) to produce a solution containing STA-conjugated particles.

(4.1.3) Third reagents (alkaline phosphatase-labeled antibody solutions)

- Third reagent for detecting PD-1

As a detection antibody, anti-PD-1 antibody (clone No. PD1.3.1.3) was used. The antibody was labeled with alkaline phosphatase using Alkaline Phosphatase Labeling Kit-SH (Catalog No. LK13, Dojindo Molecular Technologies, Inc.). The concrete operations of the procedure were carried out in accordance with the kit manual. The alkaline phosphatase-labeled anti-PD-1 antibody was purified by gel filtration. The alkaline phosphatase-labeled anti-PD-1 antibody was added to 100 mM of HEPES (pH 7.5) in such a manner that the antibody was diluted 75-fold to prepare a third reagent for detecting PD-1.

- Third reagent for detecting CTLA-4

As a detection antibody, an anti-CTLA-4 antibody (clone No. 14D3) was used. The antibody was labeled with alkaline phosphatase and was then purified in the same manner as mentioned above. The alkaline phosphatase-labeled anti-CTLA-4 antibody was added to 100 mM of MES (pH 6.5) in such a manner that the antibody was diluted 75 fold to prepare a third reagent for detecting CTLA-4.

- Third reagent for detecting PD-L1

As a detection antibody, an anti-PD-L1 antibody (clone No. 9L814) was used. The antibody was labeled with alkaline phosphatase and was then purified in the same manner as mentioned above. The alkaline phosphatase-labeled anti-PD-L1 antibody was added to 100 mM of MES (pH 6.5) in such a manner that the antibody was diluted 75-fold to prepare a third reagent for detecting PD-L1.

(4.1.4) Fourth reagent (buffer solution for measurement use)

HISCL R4 reagent (Sysmex Corporation) was used as a fourth reagent.

(4.1.5) Fifth reagent (substrate solution)

HISCL R5 reagent (Sysmex Corporation) containing CDP-Star (registered tradename) (Applied Biosystems) as a chemiluminescent substrate for alkaline phosphatase was used as a fifth reagent.

(4.2) Measurement

The measurement of each of the free protein markers was carried out with a fully automated immunoassay system HISCL-800 (Sysmex Corporation) using the first to fifth reagents. The measurement was based on sandwich ELISA on magnetic particles. Concrete operations were as follows. Plasma (20 μL) was added to and mixed with the first reagent (50 μL), and the second reagent (30 μL) was further added to and mixed with the resultant mixture. The magnetic particles in the mixed solution were magnetically collected, and a supernatant was removed. A HISCL wash solution (300 μL) was added to the resultant product to wash the magnetic particles. The supernatant was removed, and the third reagent was added to and mixed with the magnetic particles. The amounts of the third reagents added were as follows. The third reagent for detecting PD-1: 100μL, the third reagent for detecting CTLA-4: 100 μL; and the third reagent for detecting PD-L1: 80 μL. The magnetic particles in the mixed solution were magnetically collected, and a supernatant was removed. A HISCL wash solution (300 μL) was added to the resultant product to wash the magnetic particles. The supernatant was removed, and the fourth reagent (50 μL) and the fifth reagent (100 μL) were added to the magnetic particles, then the resultant mixture was fully mixed, and then the chemiluminescence intensity was measured. The reaction time was 17 minutes for the whole process. The chemiluminescence intensity was assigned to a calibration curve to calculate the concentration of each of the free protein markers.

4. Results

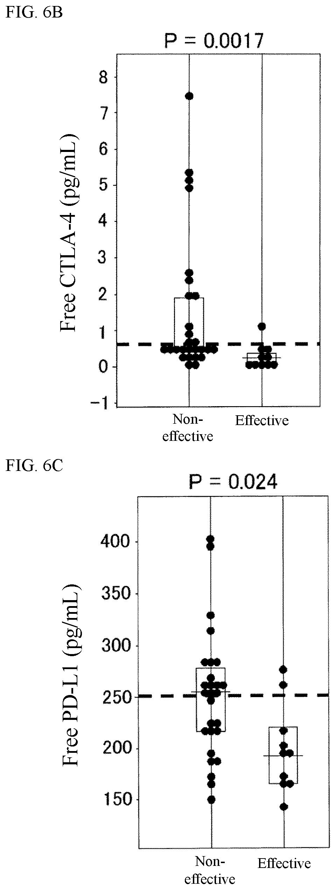

In Example 1, patients each of whom the PFS was 6 months or longer were classified into a nivolumab effective group, and patients each of whom the PFS was shorter than 6 months were classified into a nivolumab non-effective group. Among all of the patients (50 cases), the number of patients each of whom the PFS was 6 months or longer was 21 cases, and the number of patients each of whom the PFS was shorter than 6 months was 29 cases. Among the PD-L1-negative cases (37 cases), the number of patients each of whom the PFS was 6 months or longer was 10 cases, and the number of patients each of whom the PFS was shorter than 6 months was 27 cases. In Figs. 6A to 6C, the distributions of the measurement values (pg/mL) for the free protein markers in plasma before the administration of nivolumab in the PD-L1-negative cases are respectively shown. In the drawings, "Effective" indicates a group in which nivolumab was effective and "Non-effective" indicates a group in which nivolumab was not effective. In each of the drawings, a broken line indicates a median of the measurement values. As shown in Figs. 6A to 6C, it was demonstrated that, in the patients in each of whom nivolumab was effective, the measurement values for free PD-1, free CTLA-4 and free PD-L1 in blood samples tended to be lower.

The PD-L1-negative cases (37 cases) were classified in accordance with the measurement values for each of the free protein markers and the presence or absence of "PFS of 6 months". The sensitivity and the degree of specificity when the efficacy of nivolumab was determined based on the measurement values for the markers were also calculated. The results are shown in Tables 1 to 3. In the tables, with respect to "PFS of 6 months", subjects each of whom the PFS was 6 months or longer were classified into a "+" group and subjects each of whom the PFS was shorter than 6 months were classified into a "-" group. In the tables, with respect to "sPD-1", "sCTLA-4" and "sPD-L1", subjects each of whom the measurement value was lower than a threshold value were classified into a "+" group and subjects each of whom the measurement value was larger than the threshold value were classified into a "-" group. In Example 1, the threshold value corresponding to sPD-1 was 150 pg/mL, the threshold value corresponding to sCTLA-4 was 0.5 pg/mL, and the threshold value corresponding to sPD-L1 was 219 pg/mL.

[Table 1]

| | | PFS of 6 months | | Total | | | |
|---|---|---|---|---|---|---|---|
| | | + | - | | | | |
| sPD-1 | + (<150 pg/mL) | 6 | 8 | 14 | | Sensitivity | 60.0% |
| | - (>150 pg/mL) | 4 | 19 | 23 | | Degree of specificity | 70.4% |
| Total | | 10 | 27 | 37 | | | |

[Table 2]

| | | PFS of 6 months | | Total | | | |
|---|---|---|---|---|---|---|---|
| | | + | - | | | | |
| sCTLA-4 | + (<0.5 pg/mL) | 9 | 12 | 21 | | Sensitivity | 90.0% |
| | - (>0.5 pg/mL) | 1 | 15 | 16 | | Degree of specificity | 55.6% |
| Total | | 10 | 27 | 37 | | | |

[Table 3]

| | | PFS of 6 months | | Total | | | |
|---|---|---|---|---|---|---|---|
| | | + | - | | | | |
| sPD-L1 | + (<219 pg/mL) | 7 | 8 | 15 | | Sensitivity | 70.0% |
| | - (>219 pg/mL) | 3 | 19 | 22 | | Degree of specificity | 70.4% |
| Total | | 10 | 27 | 37 | | | |

The 37 patients who were classified into the "PD-L1-negative cases" were patients who were determined that nivolumab was not effective by the conventional prediction method based on immunohistochemical staining. However, in this example, six patients was further extracted as patients for whom nivolumab was effective among from the PD-L1-negative cases by the determination employing the measurement values for sPD-1. In addition, 9 patients and 7 patients were further extracted as patients for whom nivolumab was effective by the determination employing the measurement values for sCTLA-4 and sPD-L1, respectively.

Example 2

The determination accuracy of the case where the efficacy of an immune checkpoint inhibitor was determined based on the results of Example 1 was confirmed.

1. Determination of efficacy by immunohistochemical staining

The sensitivity and the degree of specificity in the case where the efficacy of nivolumab was determined based on the TPSs calculated in Example 1 were calculated. The results are shown in Table 4. In the tables, with respect to "PFS of 6 months", subjects each of whom the PFS was 6 months or longer were classified into a "+" group and subjects each of whom the PFS was shorter than 6 months were classified into a "-" group. With respect to "immunohistochemical staining (PD-L1)", subjects for each of whom the TPS was 50% or more were classified into a "+" group and subjects for each of whom the TPS was less than 50% were classified into a "-" group.

[Table 4]

| | | PFS of 6 months | | Total | | | |
|---|---|---|---|---|---|---|---|
| | | + | - | | | | |
| Immunohi stochemical staining (PD-L1) | + (≥ 50%) | 10 | 3 | 13 | | Sensitivity | 50.0% |
| | - (< 50%) | 10 | 27 | 37 | | Degree of specificity | 90.0% |
| Total | | 20 | 30 | 50 | | | |

As shown in Table 1, it was demonstrated that, in the determination based on the PD-L1 expression ratios in tumor tissues, the degree of specificity was high while the sensitivity was poor. Namely, in this determination, patients who normally responds to the immune checkpoint inhibitor were missed. Then, hereinbelow, subjects who were determined as "negative" in the immunohistochemical staining (TPS < 50%) were further subjected to the determination based on the measurement values for free marker proteins.

2. Determination of efficacy by immunohistochemical staining and free protein markers

(2.1) Rating of measurement values for free protein markers with scores

The measurement values for free protein markers (sPD-1 and sCTLA-4) in blood samples in the PD-L1-negative cases (37 cases) in Example 1 were rated with scores based on threshold values for the free protein markers. In Example 2, a median of measurement values was employed as a threshold value. As shown in Table 5, when both of the measurement value for sPD-1 and the measurement value for sCTLA-4 were respectively lower than the threshold values corresponding to the markers, the score was rated as "0". When either one of the measurement value for sPD-1 or the measurement value for sCTLA-4 was lower than the threshold value corresponding to the marker, the score was rated as "1". When both of the measurement value for sPD-1 and the measurement value for sCTLA-4 were respectively higher than the threshold values corresponding to these markers, the score was rated as "2".

[Table 5]

| sPD-1 (Threshold value: 168 pg/mL) | sCTLA-4 (Threshold value: 0.46 pg/mL) | Score |
|---|---|---|
| - | - | 0 |
| + | - | 1 |
| - | + | |
| + | + | 2 |

(2.2) Determination of efficacy

The PD-L1-negative cases (37 cases) were classified in accordance with the scores for the free protein markers and the presence or absence of "PFS of 6 months". In addition, the sensitivity and the degree of specificity in the case where the efficacy of nivolumab was determined based on the scores were calculated. The results are shown in Table 6.

[Table 6]

| | | PFS of 6 months | | Total | | | |
|---|---|---|---|---|---|---|---|
| | | + | - | | | | |
| Score of markers (sPD-1 and sCTLA-4) in blood | 0 | 8 | 7 | 15 | | Sensitivity | 80.0% |
| | 1 or 2 | 2 | 20 | 22 | | Degree of specificity | 74.1% |
| Total | | 10 | 27 | 37 | | | |

As shown in Table 6, it was demonstrated that, in the determination based on the measurement values for the free protein markers, patients for whom nivolumab was effective were selected with high sensitivity when each of the scores was rated as "0" or "1 or more". Therefore, in the determination based on the measurement values for free protein markers, it is determined that an immune checkpoint inhibitor is more likely to be effective for subjects when the score of each of the subjects is "0" and the subjects are selected as candidates for the administration of the immune checkpoint inhibitor. On the other hand, when the score of each of subjects is "1 or more", it is determined that an immune checkpoint inhibitor is less likely to be effective for the subjects and the subjects are not selected as candidates for the administration of the immune checkpoint inhibitor.

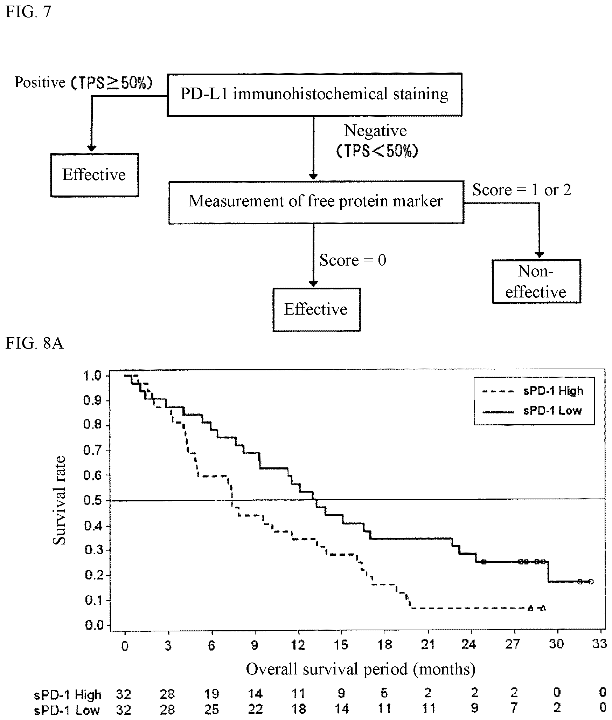

The efficacy of nivolumab was determined by employing the combination of the determination by immunohistochemical staining mentioned in section (2.1) and the determination based on measurement values for the free protein markers. The flow chart of the determination employing this combination is shown in Fig. 7. The sensitivity and the degree of specificity of the determination were calculated. The results are shown in Table 7. In the table, in "Immunohistochemical staining (PD-L1) and blood marker test (sPD-1 and sCTLA-4)", subject each of whom the TPS was 50% or more and subjects each of whom the TPS was less than 50% and the score was "0" were classified into a "+" group, and the remaining subjects were classified into a "-" group.

[Table 7]

| | | PFS of 6 months | | Total | | | |
|---|---|---|---|---|---|---|---|
| | | + | - | | | | |
| Immunohi stochemi cal staining (PD-L1) and blood marker test (sPD-1 and sCTLA-4) | + | 18 | 9 | 27 | | Sensitivity | 90.0% |
| | - | 2 | 21 | 23 | | Degree of specificity | 70.0% |
| Total | | 20 | 30 | 50 | | | |

As shown in Table 7, when the efficacy of nivolumab was determined by employing the combination of the determination by immunohistochemical staining and the determination based on the measurement values for the free protein markers, the sensitivity was significantly improved. It was demonstrated that, when a subject who gave negative results in the immunohistochemical staining was subjected to the determination based on the measurement values for the free protein markers in blood samples as shown in the flow illustrated in Fig. 7, it becomes possible to extract patients for whom the immune checkpoint inhibitor may be effective.

Example 3

With respect to the same subjects (50 cases) as those employed in Example 1, the efficacy of nivolumab was determined based on measurement values for three markers, i.e., sPD-1, sCTLA-4 and sPD-L1. The efficacy of nivolumab was also determined by employing the combination of the determination based on measurement values for the three markers and the determination by immunohistochemical staining as mentioned in Example 2.

1. Determination based on measurement values for markers

(1.1) Rating of measurement values with scores

A measurement value for each of the markers was rated with a score based on a threshold value for the marker. In Example 3, the same threshold values as those employed in Example 1 were employed as the threshold values corresponding to the markers. As shown in Table 8, when all of the measurement value for sPD-1, the measurement value for sCTLA-4 and the measurement value for sPD-L1 were respectively lower than the threshold values corresponding to the markers, the score was rated as "0". When either one of the measurement value for sPD-1, the measurement value for sCTLA-4 or the measurement value for sPD-L1 was larger than a threshold value corresponding to the marker, the score was rated as "1". When either two of the measurement value for sPD-1, the measurement value for sCTLA-4 or the measurement value for sPD-L1 were larger than threshold values corresponding to the markers, the score was rated as "2". When all of the measurement value for sPD-1, the measurement value for sCTLA-4 and the measurement value for sPD-L1 were respectively larger than threshold values corresponding to the markers, the score was rated as "3".

[Table 8]

| sPD-1 (Threshold value: 150 pg/mL) | sCTLA-4 (Threshold value: 0.5 pg/mL) | sPD-L1 (Threshold value: 219 pg/mL) | Score |
|---|---|---|---|
| - | - | - | 0 |
| + | - | - | 1 |
| - | + | - | |
| - | - | + | |
| + | + | - | 2 |
| + | - | + | |
| - | + | + | |
| + | + | + | 3 |

(1.2) Determination of efficacy

The subjects (50 cases) were classified in accordance with the scores for the free protein markers and the presence or absence of "PFS of 6 months". In addition, the sensitivity and the degree of specificity in the case where the efficacy of nivolumab was determined based on the scores were calculated. The results are shown in Table 9.

[Table 9]

| | | PFS of 6 months | | Total | | | |
|---|---|---|---|---|---|---|---|
| | | + | - | | | | |
| Score of markers in blood | 0 | 8 | 2 | 10 | | Sensitivity | 40.0% |
| | 1,2 or 3 | 12 | 28 | 40 | | Degree of specificity | 93.3% |
| Total | | 20 | 30 | 50 | | | |

As shown in Tables 9 and 4, the sensitivity and the degree of specificity of the determination based on the measurement values for the markers in blood samples were similar to those of the determination based on the immunostaining of tumor tissues. The collection of blood is less invasive compared with the collection of tumor tissues. Therefore, it was found that the determination method of the present embodiment was applicable to patients from each of whom a tumor tissue could not be excised.

2. Combination of determination based on measurement values for markers and determination by immunohistochemical staining

The efficacy of nivolumab was also determined by employing the combination of the determination based on measurement values for the three markers mentioned in section (1.2) and the determination by the immunohistochemical staining mentioned in Example 2. The results are shown in Table 10. In the table, in "Immunohistochemical staining and blood marker test", subjects each of whom the TPS was 50% or more and subjects each of whom the TPS was less than 50% and the score was "0" were classified into a "+" group, and the remaining subjects were classified into a "-" group.

[Table 10]

| | | PFS of 6 months | | Total | | | |
|---|---|---|---|---|---|---|---|
| | | + | - | | | | |
| Immunohi stochemi cal staining and blood marker test | + | 16 | 6 | 22 | | Sensitivity | 80.0% |
| | - | 4 | 24 | 28 | | Degree of specificity | 80.0% |
| Total | | 20 | 30 | 50 | | | |

As shown in Table 10, it was demonstrated that the determination having both of high sensitivity and a high degree of specificity became possible and the determination accuracy was improved by employing the combination of the determination by immunohistochemical staining and the determination based on the measurement values for the three free protein markers.

Example 4

With respect to esophageal cancer patients and endometrial cancer patients, the relationship between the concentration of a free protein marker in a blood sample before the administration of an immune checkpoint inhibitor and the therapeutic effect after the administration of the immune checkpoint inhibitor was examined.

1. Subjects

Esophageal cancer patients (64 cases) and endometrial cancer patients (22 cases) to whom an immune checkpoint inhibitor was not administered yet were selected as subjects. Nivolumab (Ono Pharmaceutical Co., Ltd.) was administered to all of the patients, and the clinical course of each of the patients was followed up by employing an overall survival (OS) period as an evaluation measure.

2. Liquid samples and measurement

Blood was collected from each of the patients before the administration of the immune checkpoint inhibitor, and plasma was prepared from the blood. The concentrations of the free protein markers were calculated using the resultant plasma samples in the same manner as in Example 1.

3. Results

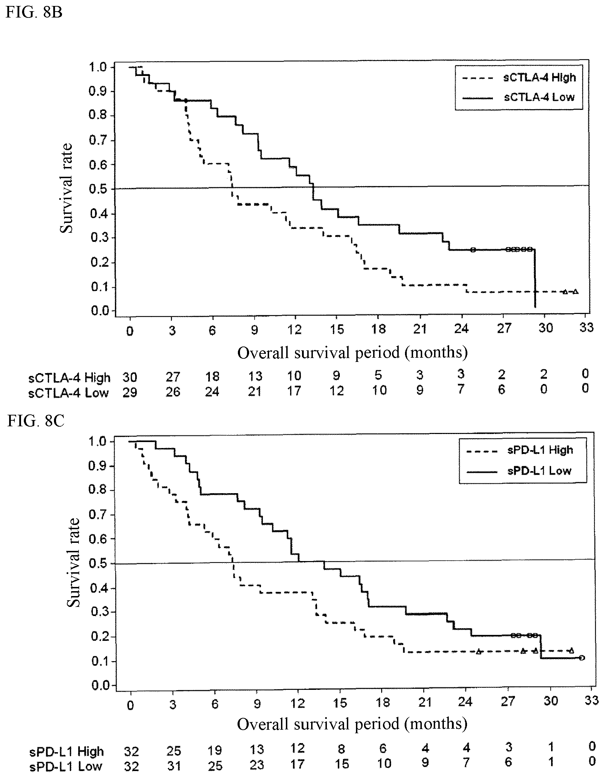

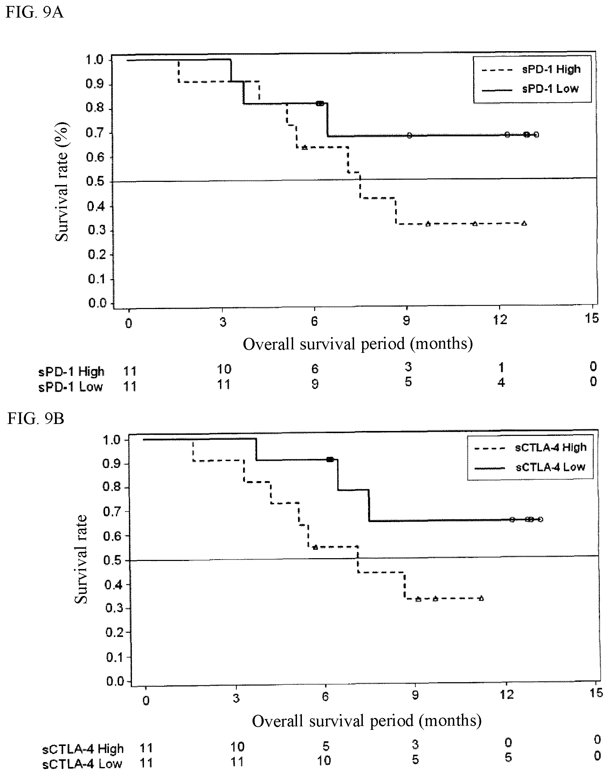

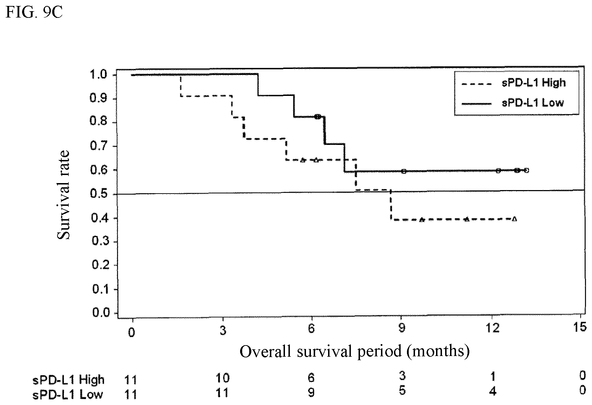

Survival rate curves were produced by Kaplan-Meier method in which the comparison was made between a group having higher measurement values and a group having smaller measurement values with respect to the free protein markers. The results are shown in Figs. 8 and 9. In each of the drawings, a horizontal line indicates a line corresponding to a survival rate of 50%. In each of the drawings, a numerical value for each of the groups indicated below the horizontal axis indicates the number of surviving patients at each of the points of time indicated on the horizontal axis. As shown in these drawings, it was demonstrated that the OS period was prolonged in a group having smaller measurement values for all of the free protein markers (the group is indicated by a broken line in each of the drawings) as the result of the administration of the immune checkpoint inhibitor compared with a group having larger measurement values for the free protein markers (the group is indicated by a solid line in each of the drawings). From these results, it was suggested that the determination method of the present embodiment was also applicable to esophageal cancer patients and endometrial cancer patients.

DESCRIPTION OF REFERENCE SIGNS

11, 21, 31: Reagent kit
12, 22, 32: First container
13, 23, 33: Second container
24, 34: Third container
25, 35: Fourth container
36: Fifth container
37: Sixth container
14, 27, 38: Packaging box
15, 26, 39: Package insert
10: Determination device
20: Immunoassay device
30: Computer system
40: Recording medium
300: Computer main body
301: Input section
302: Display section
310: CPU
311: ROM
312: RAM
313: Hard disk
314: Input/output interface
315: Reading device
316: Communication interface
317: Image output interface
318: Bus

**Claims**

1.  A method for assisting a determination of an efficacy of an immune checkpoint inhibitor, the method comprising steps of:

    measuring a free protein marker in a liquid sample collected from a subject; and
    determining the efficacy of the immune checkpoint inhibitor in the subject based on a result of the measurement, wherein
    the free protein marker is at least one selected from free Cytotoxic T lymphocyte antigen-4 (CTLA-4), free Programmed cell death-1 (PD-1) and free Programmed cell death-ligand 1 (PD-L1).

2.  The method according to claim 1, wherein the free protein markers are at least two selected from free CTLA-4, free PD-1 and free PD-L1.

3.  The method according to claim 1, wherein the free protein markers are free CTLA-4, free PD-1 and free PD-L1.

4.  The method according to any one of claims 1 to 3, wherein, in the determination step, it is determined that the immune checkpoint inhibitor is effective for the subject when a measurement value for the free protein marker which is acquired in the measurement step is lower than a predetermined threshold value corresponding to each free protein marker.

5.  The method according to claim 1, wherein
    the free protein markers are two selected from free CTLA-4, free PD-1 and free PD-L1, and
    it is determined that the immune checkpoint inhibitor is effective for the subject when a measurement value for one of the free protein markers is lower than a threshold value corresponding to the one free protein marker and a measurement value for the other free protein marker is lower than a threshold value corresponding to the other free protein marker.

6. The method according to claim 1, wherein
the free protein marker is free CTLA-4, free PD-1 and free PD-L1, and
it is determined that the immune checkpoint inhibitor is effective for the subject when a measurement value for free CTLA-4 is lower than a threshold value corresponding to free CTLA-4, a measurement value for free PD-1 is lower than a threshold value corresponding to free PD-1, and a measurement value for free PD-L1 is lower than a threshold value corresponding to free PD-L1.

7. The method according to any one of claims 1 to 6, wherein the subject is a cancer patient who is determined that the ratio of PD-L1-positive tumor cells is smaller than a predetermined value by immunostaining of a tumor tissue.

8. The method according to any one of claims 1 to 7, wherein the subject is a lung cancer patient, an esophageal cancer patient or an endometrial cancer patient.

9. The method according to any one of claims 1 to 8, wherein the immune checkpoint inhibitor is a preparation comprising an anti-PD-1 antibody as an active ingredient.

10. The method according to any one of claims 1 to 9, wherein the liquid sample is a blood sample.

11. A method for acquiring a measurement value for a free protein marker, the method comprising a step of acquiring the measurement value for the free protein marker in a liquid sample collected from a subject, wherein
the free protein marker is at least one selected from free Cytotoxic T lymphocyte antigen-4 (CTLA-4), free Programmed cell death-1 (PD-1) and free Programmed cell death-ligand 1 (PD-L1), and
it is suggested that an immune checkpoint inhibitor is effective for the subject when the acquired measurement value for the free protein marker is lower than a predetermined threshold value corresponding to each free protein marker.

12. The method according to claim 11, wherein the free protein markers are at least two selected from free CTLA-4, free PD-1 and free PD-L1.

13. The method according to claim 11, wherein the free protein markers are free CTLA-4, free PD-1 and free PD-L1.

14. The method according to any one of claims 11 to 13, wherein the immune checkpoint inhibitor is a preparation comprising an anti-PD-1 antibody as an active ingredient.

15. The method according to any one of claims 11 to 14, wherein the subject is a lung cancer patient, an esophageal cancer patient or an endometrial cancer patient.

16. The method according to any one of claims 11 to 15, wherein the liquid sample is a blood sample.

17. A method for assisting a determination of an efficacy of an immune checkpoint inhibitor, the method comprising steps of:

carrying out immunostaining of a tumor tissue collected from a subject and calculating a ratio of PD-L1-positive tumor cells;
measuring a free protein marker in a liquid sample collected from the subject; and
determining the efficacy of the immune checkpoint inhibitor in the subject based on a result of the calculation and a result of the measurement, wherein
the free protein marker is at least one selected from free Cytotoxic T lymphocyte antigen-4 (CTLA-4), free Programmed cell death-1 (PD-1) and free Programmed cell death-ligand 1 (PD-L1).

18. The method according to claim 17, wherein, in the determination step, it is determined that the immune checkpoint inhibitor is effective for the subject when the ratio of the PD-L1-positive tumor cells which has been acquired in the measurement step is smaller than a predetermined value and a measurement value for the free protein marker which is acquired in the measurement step is lower than a predetermined threshold value corresponding to each free protein marker.

19. A reagent kit for use in the method according to any one of claims 1 to 18, the reagent kit comprising at least one reagent selected from a reagent comprising a substance capable of binding specifically to free Cytotoxic T lymphocyte antigen-4 (CTLA-4), a reagent comprising a substance capable of binding specifically to free Programmed cell death-1 (PD-1) and a reagent comprising a substance capable of binding specifically to free Programmed cell death-ligand 1 (PD-L1).

20. The reagent kit according to claim 19, the reagent kit comprising at least two reagents selected from a reagent comprising a substance capable of binding specifically to free CTLA-4, a reagent comprising a substance capable of binding specifically to free PD-1 and a reagent comprising a substance capable of binding specifically to free PD-L1.

21. The reagent kit according to claim 19, the reagent kit comprising a reagent comprising a substance capable of binding specifically to free CTLA-4, a reagent comprising a substance capable of binding specifically to free PD-1 and a reagent comprising a substance capable of binding specifically to free PD-L1.

22. A device for determining an efficacy of an immune checkpoint inhibitor, the device being equipped with a computer that is equipped with a processor and a memory placed under a control of the processor, the memory recording a computer program for bringing the computer to execute the steps of:

acquiring a measurement value for a free protein marker in a liquid sample collected from a subject; and
determining the efficacy of the immune checkpoint inhibitor in the subject based on the measurement value for the free protein marker, wherein
the free protein marker is at least one selected from free Cytotoxic T lymphocyte antigen-4 (CTLA-4), free Programmed cell death-1 (PD-1) and free Programmed cell death-ligand 1 (PD-L1).

23. The device according to claim 22, wherein the free protein markers are at least two selected from free CTLA-4, free PD-1 and free PD-L1.

24. The device according to claim 22, wherein the free protein markers are free CTLA-4, free PD-1 and free PD-L1.

25. A device for acquiring a measurement value for a free protein marker, the device being equipped with a computer that is equipped with a processor and a memory placed under the control of the processor, the memory recording a computer program for bringing the computer to execute the step of acquiring a measurement value for a free protein marker in a liquid sample collected from a subject, wherein
the free protein marker is at least one selected from free Cytotoxic T lymphocyte antigen-4 (CTLA-4), free Programmed cell death-1 (PD-1) and free Programmed cell death-ligand 1 (PD-L1), and
it is suggested that an immune checkpoint inhibitor is effective for the subject when the acquired measurement value for the free protein marker is lower than a predetermined threshold value corresponding to each free protein marker.

26. The device according to claim 25, wherein the free protein markers are at least two selected from free CTLA-4, free PD-1 and free PD-L1.

27. The device according to claim 25, wherein the free protein markers are free CTLA-4, free PD-1 and free PD-L1.

28. A computer program for use in a determination of an efficacy of an immune checkpoint inhibitor, the computer program being recorded on a computer-readable medium, wherein
the computer program is a computer program for bringing the computer to execute the steps of:

acquiring a measurement value for a free protein marker in a liquid sample collected from a subject; and
determining the efficacy of the immune checkpoint inhibitor in the subject based on the measurement value for the free protein marker, and
the free protein marker is at least one selected from free Cytotoxic T lymphocyte antigen-4 (CTLA-4), free Programmed cell death-1 (PD-1) and free Programmed cell death-ligand 1 (PD-L1).

29. The computer program according to claim 28, wherein the free protein markers are at least two selected from free CTLA-4, free PD-1 and free PD-L1.

30. The computer program according to claim 28, wherein the free protein markers are free CTLA-4, free PD-1 and free PD-L1.

31. A pharmaceutical composition for cancer treatment, the pharmaceutical composition comprising an immune checkpoint inhibitor and being administered to a cancer patient who is determined that the immune checkpoint inhibitor is effective by the method according to any one of claims 1 to 10, 17 and 18.

FIG. 1A

FIG. 1B

FIG. 1C

FIG. 2

FIG. 3

FIG. 4A

```
                    ┌─────────────┐
                    │    START    │
                    └──────┬──────┘
                           │                    S101
           ┌───────────────▼───────────────┐
           │ Acquire measurement value for free │
           │             PD-1              │
           └───────────────┬───────────────┘
                           │                    S102
                    ╱──────▼──────╲
                  ╱   Measurement   ╲        No
                ◄  value for free PD-1 < first ────────────┐
                  ╲  threshold value? ╱                    │
                    ╲──────┬──────╱                        │
                       Yes │       S103                S104 │
           ┌───────────────▼───────────────┐    ┌───────────▼───────────────┐
           │ Send determination result that immune │    │ Send determination result that immune │
           │ checkpoint inhibitor is effective for │    │ checkpoint inhibitor is not effective │
           │           subject             │    │          for subject          │
           └───────────────┬───────────────┘    └───────────┬───────────────┘
                           │                                │
                           ◄────────────────────────────────┘
                           │                    S105
           ┌───────────────▼───────────────┐
           │   Output determination result   │
           └───────────────┬───────────────┘
                    ┌──────▼──────┐
                    │     END     │
                    └─────────────┘
```

FIG. 4B

```
          ┌─────────────┐
          │    START    │
          └──────┬──────┘
                 │                          S201
                 ▼
   ┌──────────────────────────────┐
   │  Acquire measurement value for free │
   │  PD-1 and measurement value for free│
   │            CTLA-4            │
   └──────────────┬───────────────┘
                  │                         S202
                  ▼
            ╱───────────╲        No
        ╱   Measurement    ╲──────────────────────┐
       ╱  value for free PD-1 < first ╲            │
        ╲    threshold value?    ╱                 │
            ╲───────────╱                          │
                  │ Yes                            │
                  │                    S203        │
                  ▼                                │
            ╱───────────╲        No                │
        ╱   Measurement    ╲────────────┐          │
       ╱ value for free CTLA-4 < second ╲          │
        ╲    threshold value?    ╱       │         │
            ╲───────────╱                │         │
                  │ Yes       S204       │         │
                  ▼                      │         │
                                         │    S205 │
   ┌──────────────────────────────┐  ┌──────────────────────────────┐
   │  Send determination result that │  │  Send determination result that │
   │  immune checkpoint inhibitor is  │  │  immune checkpoint inhibitor is  │
   │     effective for subject       │  │   not effective for subject     │
   └──────────────┬───────────────┘  └──────────────┬───────────────┘
                  │◄──────────────────────────────────┘
                  │                    S206
                  ▼
   ┌──────────────────────────────┐
   │    Output determination result  │
   └──────────────┬───────────────┘
                  │
                  ▼
          ┌─────────────┐
          │     END     │
          └─────────────┘
```

FIG. 4C

```
                    ┌─────────────┐
                    │    START    │
                    └──────┬──────┘
                           │                      S301
        ┌──────────────────▼──────────────────┐
        │ Acquire measurement value for free PD-1,│
        │ measurement value for free CTLA-4 and   │
        │ measurement value for free PD-L1        │
        └──────────────────┬──────────────────┘
                           │                      S302
                      ╱────▼────╲
                    ╱ Measurement ╲      No
                   ╱ value for free  ╲──────────────────────┐
                   ╲ PD-1 < first    ╱                      │
                    ╲ threshold value? ╱                     │
                      ╲────┬────╱                            │
                        Yes │                     S303       │
                      ╱────▼────╲                            │
                    ╱ Measurement ╲      No                  │
                   ╱ value for free  ╲──────────────────────┤
                   ╲ CTLA-4 < second ╱                       │
                    ╲ threshold value? ╱                      │
                      ╲────┬────╱                            │
                        Yes │                     S304       │
                      ╱────▼────╲                            │
                    ╱ Measurement ╲      No                  │
                   ╱ value for free  ╲──────────────────────┤
                   ╲ PD-L1 < third   ╱                       │
                    ╲ threshold value? ╱                      │
                      ╲────┬────╱                            │
                        Yes │          S305      S306        │
        ┌──────────────────▼──────────┐  ┌──────────────────▼──────────┐
        │ Send determination result    │  │ Send determination result    │
        │ that immune checkpoint        │  │ that immune checkpoint        │
        │ inhibitor is effective for    │  │ inhibitor is not effective for│
        │ subject                       │  │ subject                       │
        └──────────────────┬──────────┘  └──────────────────┬──────────┘
                           │◄──────────────────────────────┘
                           │                      S307
        ┌──────────────────▼──────────┐
        │ Output determination result  │
        └──────────────────┬──────────┘
                    ┌──────▼──────┐
                    │     END     │
                    └─────────────┘
```

FIG. 5

FIG. 6A

FIG. 6B

FIG. 6C

FIG. 7

Positive (TPS≥50%)

PD-L1 immunohistochemical staining

Negative
(TPS<50%)

Effective

Measurement of free protein marker

Score = 1 or 2

Score = 0

Non-effective

Effective

FIG. 8A

| | | | | | | | | | | | | |
|---|---|---|---|---|---|---|---|---|---|---|---|---|
| sPD-1 High | 32 | 28 | 19 | 14 | 11 | 9 | 5 | 2 | 2 | 2 | 0 | 0 |
| sPD-1 Low | 32 | 28 | 25 | 22 | 18 | 14 | 11 | 11 | 9 | 7 | 2 | 0 |

FIG. 8B

| | 0 | 3 | 6 | 9 | 12 | 15 | 18 | 21 | 24 | 27 | 30 | 33 |
|---|---|---|---|---|---|---|---|---|---|---|---|---|
| sCTLA-4 High | 30 | 27 | 18 | 13 | 10 | 9 | 5 | 3 | 3 | 2 | 2 | 0 |
| sCTLA-4 Low | 29 | 26 | 24 | 21 | 17 | 12 | 10 | 9 | 7 | 6 | 0 | 0 |

FIG. 8C

| | 0 | 3 | 6 | 9 | 12 | 15 | 18 | 21 | 24 | 27 | 30 | 33 |
|---|---|---|---|---|---|---|---|---|---|---|---|---|
| sPD-L1 High | 32 | 25 | 19 | 13 | 12 | 8 | 6 | 4 | 4 | 3 | 1 | 0 |
| sPD-L1 Low | 32 | 31 | 25 | 23 | 17 | 15 | 10 | 9 | 7 | 6 | 1 | 0 |

FIG. 9A

| | | | | | |
|---|---|---|---|---|---|
| sPD-1 High | 11 | 10 | 6 | 3 | 1 | 0 |
| sPD-1 Low | 11 | 11 | 9 | 5 | 4 | 0 |

FIG. 9B

| | | | | | |
|---|---|---|---|---|---|
| sCTLA-4 High | 11 | 10 | 5 | 3 | 0 | 0 |
| sCTLA-4 Low | 11 | 11 | 10 | 5 | 5 | 0 |

FIG. 9C

| | sPD-L1 High | sPD-L1 Low |
Survival rate vs Overall survival period (months)

| | | | | | |
|---|---|---|---|---|---|
| sPD-L1 High | 11 | 10 | 6 | 3 | 1 | 0 |
| sPD-L1 Low | 11 | 11 | 9 | 5 | 4 | 0 |

## INTERNATIONAL SEARCH REPORT

| International application No. |
|---|
| PCT/JP2019/010601 |

### A. CLASSIFICATION OF SUBJECT MATTER
Int.Cl. G01N33/68(2006.01)i, A61K39/395(2006.01)i, A61K45/00(2006.01)i, A61P35/00(2006.01)i, A61P37/04(2006.01)i, G01N33/53(2006.01)i, G01N33/574(2006.01)i

According to International Patent Classification (IPC) or to both national classification and IPC

### B. FIELDS SEARCHED

Minimum documentation searched (classification system followed by classification symbols)
Int.Cl. G01N33/68, A61K39/395, A61K45/00, A61P35/00, A61P37/04, G01N33/53, G01N33/574

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | YUASA, Takeshi et al., "Biomarkers to predict prognosis and response to checkpoint inhibitors", Int. J. Clin. Oncol., August 2017, vol. 22, no. 4, pp. 629-634 | 1-31 |
| A | 北野滋久, 免疫チェックポイント阻害療法におけるバイオマーカー, カレントテラピー, 01 February 2017, vol. 35, no. 2, pp. 156-160, non-official translation (KITANO, Shigehisa, "Biomarker in immune checkpoint inhibitor therapy", CURRENT THERAPY) | 1-31 |
| A | 各務博, チェックポイント阻害剤のバイオマーカー, 呼吸器内科, 28 December 2017, vol. 32, no. 6, pp. 589-595, (KAGAMU, Hiroshi, "Biomarkers to predict antitumor reactivity of immune checkpoint inhibitors", Respiratory medicine) | 1-31 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 June 2019 (04.06.2019) | 18 June 2019 (18.06.2019) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2019/010601

C (Continuation).    DOCUMENTS CONSIDERED TO BE RELEVANT

| Category* | Citation of document, with indication, where appropriate, of the relevant passages | Relevant to claim No. |
|---|---|---|
| A | 大塚篤司，5.悪性黒色腫の PD-1 阻害がん免疫治療と治療効果予測バイオマーカー，血液フロンテティア，30 October 2017, vol. 27, no. 11, pp. 1547-1554, non-official translation (OTSUKA, Atsushi, "PD-1 inhibitor cancer immunotherapy for malignant melanoma, and biomarker for predicting therapeutic effect thereof", Hematology Frontier) | 1-31 |

Form PCT/ISA/210 (continuation of second sheet) (January 2015)

<table>
<tr><td>**INTERNATIONAL SEARCH REPORT**</td><td>International application No.<br>PCT/JP2019/010601</td></tr>
</table>

**Box No. II     Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:
See extra sheet

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

| International application No. |
| --- |
| PCT/JP2019/010601 |

```
<Continuation of Box No. III>
```

Claims 1-31 have the common technical feature of [measuring a free protein marker in a liquid sample obtained from a subject, and determining efficacy of an immune checkpoint inhibitor]. However, since said technical feature does not make a contribution over the prior art in light of the disclosure of document 1, this technical feature cannot be considered a special technical feature. Therefore, claims 1-31 are considered to describe a different invention each independently with respect to three different biomarkers, and there are no other identical or corresponding special technical features between these inventions. Accordingly, the claims are classified into three inventions respectively having the following special technical features.

(Invention 1) Claims 1-31 (pars thereof)
Invention with respect to a free CTLA-4 (Cytotoxic T lymphocyte antigen-4) as a biomarker for determining efficacy of an immune checkpoint inhibitor.
(Invention 2) Claims 1-31 (pars thereof)
Invention with respect to a free PD-1 (Programmed cell death-1) as a biomarker for determining efficacy of an immune checkpoint inhibitor.
(Invention 3) Claims 1-31 (pars thereof)
Invention with respect to a free PD-L1 (Programmed cell death-ligand 1) as a biomarker for determining efficacy of an immune checkpoint inhibitor.

Document 1: YUASA, Takeshi et al., "Biomarkers to predict prognosis and response to checkpoint inhibitors", Int. J. Clin. Oncol., August 2017, vol. 22, no. 4, pp. 629-634

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HI254868 B

**Non-patent literature cited in the description**

- **AGATA.** Expression of the PD-1 antigen on the surface of stimulated mouse T and B lymphocytes. *Int. Immunol.,* 1996, vol. 8, 765-772